# EUROPÄISCHE PATENTSCHRIFT

(11) **EP 2 560 986 B1**
(45) Veröffentlichungstag und Bekanntmachung des Hinweises auf die Patenterteilung: **02.06.2021**
(21) Anmeldenummer: 11718679.1
(22) Anmeldetag: 20.04.2011
(51) Int. Cl.: C12N 15/85

(54) **GENETISCH MODIFIZIERTES SENDAIVIRUS ZUR BEHANDLUNG VON TUMORERKRANKUNGEN**
GENETICALLY MODIFIED SENDAIVIRUS FOR THE TREATMENT OF TUMOURS
VIRUS SENDAI GÉNÉTIQUEMENT MODIFIÉ POUR LE TRAITEMENT DE TUMEURS

(30) Priorität: 23.04.2010 DE 102010018961
(43) Veröffentlichungstag der Anmeldung: 27.02.2013
(73) Patentinhaber: Eberhard-Karls-Universität Tübingen Universitätsklinikum, 72076 Tübingen (DE)
(72) Erfinder: LAUER, Ulrich Manfred, 72072 Tuebingen (DE); BITZER, Michael, 72108 Rottenburg (DE); ZIMMERMANN, Martina, 72072 Tuebingen (DE); ARMEANU-EBINGER, Sorin, 72076 Tuebingen (DE); BOSSOW, Sascha, 69115 Heidelberg (DE); NEUBERT, Wolfgang, 86926 Greifenberg (DE)
(74) Vertreter: Witte, Weller & Partner Patentanwälte mbB
(86) Internationale Anmeldenummer: PCT/EP2011/056290
(87) Internationale Veröffentlichungsnummer: WO 2011/131703

(56) Entgegenhaltungen:
- EP-A1- 1 505 154
- US-A1- 2009 175 826
- OLDONI ET AL: "The use of in situ hybridization and immunohistochemistry to study the pathogenesis of various Newcastle disease virus strains and recombinants in embryonated chicken eggs", MICROBIAL PATHOGENESIS, ACADEMIC PRESS LIMITED, NEW YORK, NY, US, Bd. 39, Nr. 3, 1. September 2005 (2005-09-01), Seiten 69-75, XP005067226, ISSN: 0882-4010, DOI: DOI:10.1016/J.MICPATH.2005.04.002
- KINOH H ET AL: "Generation of a recombinant Sendai virus that is selectively activated and lyses human tumor cells expressing matrix metalloproteinases.", GENE THERAPY, Bd. 11, Nr. 14, Juli 2004 (2004-07), Seiten 1137-1145, XP002653218, ISSN: 0969-7128 in der Anmeldung erwähnt
- VAHA-KOSKELA ET AL: "Oncolytic viruses in cancer therapy", CANCER LETTERS, NEW YORK, NY, US, Bd. 254, Nr. 2, 8. September 2007 (2007-09-08), Seiten 178-216, XP022182282, ISSN: 0304-3835, DOI: DOI:10.1016/J.CANLET.2007.02.002
- KATO A ET AL: "THE PARAMYXOVIRUS, SENDAI VIRUS, V PROTEIN ENCODES A LUXURY FUNCTION REQUIRED FOR VIRAL PATHOGENESIS", EMBO JOURNAL, OXFORD UNIVERSITY PRESS, SURREY, GB, vol. 16, 1 January 1997 (1997-01-01), pages 578-587, XP000941467, ISSN: 0261-4189, DOI: 10.1093/EMBOJ/16.3.578

### SEQUENZPROTOKOLL

<110> Eberhard-Karls-Universität Tübingen, Universitätsklinikum
<120> Genetisch modifiziertes Paramyxovirus zur Behandlung von Tumorerkrankungen
<130> 5402P453
<160> 34
<170> PatentIn Version 3.3
<210> 1
   <211> 1698
   <212> DNA/RNA
   <213> Sendai Virus
<400> 1
   atggcgactt atatccagag ggtacagtgc atatctgcat tattgtcagt tgtgctcaca      60
   acactggtct cgtgtcaaat tcctagagat aggctctcta atataggggt catagtcgac     120
   gaagggaaat cactgaagat tgccgggtcc cacgaatcta ggtacatcgt attaagtctg     180
   gtcccaggga tagacctcga aaatgggtgc ggaacagctc aggttatcca gtataagagc     240
   ctactgaacc ggttgttaat cccattgaga gatgccttag atcttcagga ggctctgata     300
   actgtcacta atgacacgat gaccggtgct gatgtcccac agtcaagatt cttcggcgcc     360
   gtgattggga ccatagcact tggagtggca acatcagcac agataaccgc agggatcgcg     420
   ctagctgagg cgagggaggc caagagagac atagcactca taaaagaatc aatgacaaag     480
   acacacaaat ctatagaact gctgcaaaac gctgtggggg aacaaattct tgctctaaag     540
   acgctccagg atttcgtgaa tgatgagatc aaacccgcga taagcgaatt gggttgtgag     600
   actgccgctt taagactggg gataaaactg acacaacatt actccgagct gctgactgca     660
   tttggatcta actttggaac catcggagag aagagcctca cactgcaggc actgtcttca     720
   ctctactctg ctaacatcac tgagattatg accacgatca ggacagggca gtccaacatc     780
   tatgacgtca tttatacgga gcagatcaag ggaactgtga ttgatgtaga tctagagaga     840
   tacatggtta ccctgtctgt aaaaatcccc attctttctg aagtcccagg tgtgcttata     900
   cacaaagcat cgtctatttc ttacaatata gacggggagg agtggtatgt gactgtcccc     960
   agtcacatac tcagtcgtgc ctccttcctg gggggtgcaa acatagctga ttgtgtagag    1020
   tccagattga cctacatatg ccctagggat cctgcacaat tgatacctga cagccagcaa    1080
   aaatgtatct tgggggacac aacaaggtgt cctgtcacaa aggttgtgga taacatcatc    1140
   cccaaatttg cctttgtaaa tggaggtgtc gttgcgaact gcatagcatc cacatgtacc    1200
   tgtgggacag gccgaaggcc aatcagtcag gatcgctcta aaggtgtagt attcttaact    1260
   catgataact gtggactcat aggagtcaat gggatagaat tgtatgctaa taggaaaggg    1320
   catgatgcca cttggggggt ccagaatttg acagtcggtc ctgcaatcgc tatcagaccc     1380
   gtggacattt ctctcaacct tgccgctgct actgacttcc tgcaagactc tagagctgaa    1440
   cttgagaagg cacgaaaaat cctctctgaa gtaggtagat ggtacaattc aggggcgact    1500
   ttgattacga tcatagtagt catgattgta gtattggtgg tcattatagt gattgtcatt    1560
   gtgctctaca gactcaggag atcaatgcta atgagtaatc cagccggtcg gatatcaaga    1620
   gacacatata cattagagcc gaagataagg catatgtata ctaacggtgg gttcgatgct    1680
   atgactgaga aaagatag                                                  1698
<210> 2
   <211> 565
   <212> PRT
   <213> Sendai Virus
<400> 2
   Met Ala Thr Tyr Ile Gln Arg Val Gln Cys Ile Ser Ala Leu Leu Ser
   1               5                   10                  15
   Val Val Leu Thr Thr Leu Val Ser Cys Gln Ile Pro Arg Asp Arg Leu
               20                  25                  30
   Ser Asn Ile Gly Val Ile Val Asp Glu Gly Lys Ser Leu Lys Ile Ala
           35                  40                  45
   Gly Ser His Glu Ser Arg Tyr Ile Val Leu Ser Leu Val Pro Gly Ile
       50                  55                  60
   Asp Leu Glu Asn Gly Cys Gly Thr Ala Gln Val Ile Gln Tyr Lys Ser
   65                  70                  75                  80
   Leu Leu Asn Arg Leu Leu Ile Pro Leu Arg Asp Ala Leu Asp Leu Gln
                   85                  90                  95
   Glu Ala Leu Ile Thr Val Thr Asn Asp Thr Met Thr Gly Ala Asp Val
               100                 105                 110
   Pro Gln Ser Arg Phe Phe Gly Ala Val Ile Gly Thr Ile Ala Leu Gly
           115                 120                 125
   Val Ala Thr Ser Ala Gln Ile Thr Ala Gly Ile Ala Leu Ala Glu Ala
       130                 135                 140
   Arg Glu Ala Lys Arg Asp Ile Ala Leu Ile Lys Glu Ser Met Thr Lys
   145                 150                 155                 160
   Thr His Lys Ser Ile Glu Leu Leu Gln Asn Ala Val Gly Glu Gln Ile
                   165                 170                 175
   Leu Ala Leu Lys Thr Leu Gln Asp Phe Val Asn Asp Glu Ile Lys Pro
               180                 185                 190
   Ala Ile Ser Glu Leu Gly Cys Glu Thr Ala Ala Leu Arg Leu Gly Ile
           195                 200                 205
   Lys Leu Thr Gln His Tyr Ser Glu Leu Leu Thr Ala Phe Gly Ser Asn
       210                 215                 220
   Phe Gly Thr Ile Gly Glu Lys Ser Leu Thr Leu Gln Ala Leu Ser Ser
   225                 230                 235                 240
   Leu Tyr Ser Ala Asn Ile Thr Glu Ile Met Thr Thr Ile Arg Thr Gly
                   245                 250                 255
   Gln Ser Asn Ile Tyr Asp Val Ile Tyr Thr Glu Gln Ile Lys Gly Thr
               260                 265                 270
   Val Ile Asp Val Asp Leu Glu Arg Tyr Met Val Thr Leu Ser Val Lys
           275                 280                 285
   Ile Pro Ile Leu Ser Glu Val Pro Gly Val Leu Ile His Lys Ala Ser
       290                 295                 300
   Ser Ile Ser Tyr Asn Ile Asp Gly Glu Glu Trp Tyr Val Thr Val Pro
   305                 310                 315                 320
   Ser His Ile Leu Ser Arg Ala Ser Phe Leu Gly Gly Ala Asn Ile Ala
                   325                 330                 335
   Asp Cys Val Glu Ser Arg Leu Thr Tyr Ile Cys Pro Arg Asp Pro Ala
               340                 345                 350
   Gln Leu Ile Pro Asp Ser Gln Gln Lys Cys Ile Leu Gly Asp Thr Thr
           355                 360                 365
   Arg Cys Pro Val Thr Lys Val Val Asp Asn Ile Ile Pro Lys Phe Ala
       370                 375                 380
   Phe Val Asn Gly Gly Val Val Ala Asn Cys Ile Ala Ser Thr Cys Thr
   385                 390                 395                 400
   Cys Gly Thr Gly Arg Arg Pro Ile Ser Gln Asp Arg Ser Lys Gly Val
                   405                 410                 415
   Val Phe Leu Thr His Asp Asn Cys Gly Leu Ile Gly Val Asn Gly Ile
               420                 425                 430
   Glu Leu Tyr Ala Asn Arg Lys Gly His Asp Ala Thr Trp Gly Val Gln
                   435                 440                 445
   Asn Leu Thr Val Gly Pro Ala Ile Ala Ile Arg Pro Val Asp Ile Ser
       450                 455                 460
   Leu Asn Leu Ala Ala Ala Thr Asp Phe Leu Gln Asp Ser Arg Ala Glu
   465                 470                 475                 480
   Leu Glu Lys Ala Arg Lys Ile Leu Ser Glu Val Gly Arg Trp Tyr Asn
                   485                 490                 495
   Ser Gly Ala Thr Leu Ile Thr Ile Ile Val Val Met Ile Val Val Leu
               500                 505                 510
   Val Val Ile Ile Val Ile Val Ile Val Leu Tyr Arg Leu Arg Arg Ser
           515                 520                 525
   Met Leu Met Ser Asn Pro Ala Gly Arg Ile Ser Arg Asp Thr Tyr Thr
       530                 535                 540
   Leu Glu Pro Lys Ile Arg His Met Tyr Thr Asn Gly Gly Phe Asp Ala
   545                 550                 555                 560
   Met Thr Glu Lys Arg
                   565
<210> 3
   <211> 1707
   <212> DNA/RNA
   <213> Sendai Virus
<400> 3
   atggatcaag atgccctcat ttctaaagaa gattctgaag ttgagaggga ggcgtcagga      60
   ggacgagagt cgctctcgga tgttatcgga ttcctcgacg cagtcctatc aagtgaacca     120
   actgacatcg gaggggacag aagctggctc cacaatacca tcaacaccct ccaaaggcca     180
   ggctctaccc acagagtcaa aggtgagggc gagggagaag tctcgacatc gtcgacccaa     240
   gataatcgat caggtgagga gagtagagtc tctgggggaa caagcgagcc agaggcagaa     300
   gcacatgcta gaaacgttga taaacaaaat atacactggg ccactgggag aggagctagt     360
   acagactctg tacctcagga tctgggcaat ggaagagact ccggaatcct tgaagatcct     420
   ccaaatgagg gaggatatcc gagatcaggt gctgaagatg aaaaccgaga gatggctgcg     480
   aaccctgata agaggggaga agaccaagct gaaggacttc cagaagagat acgaagaagt     540
   gcacccctac ctgatgaaag agaaggtaga gcagataata atggaagagg cgtggagcct     600
   ggcagcccac atagtgcaag agtgactgga gtcttggtga tccccagtcc tgagctcgaa     660
   gaggccgtgc tacaaaggaa caagagacga cccgccaaca gcgggtccag atctctcacc     720
   ccagtggtcg tgcctagcac tcggtctcca ccaccggacc atgacaatag tacaaggtca     780
   ccaccaagga aacccccaac cacgcaggat gagcacacca accccaggaa cacccccgcc     840
   gtcaggatca aggatcggag acccccaaca gggactcgct ccgccccaga ccgcccgacc     900
   gacggctatc caacccaccc aagtccagag accgatgcaa caaaaaaggg catagaagag     960
   aacacatcat ctatgaaaga gatggctaca ttgttaacga gtcttggtgt aatccagtct    1020
   gctcaagaat tcgagtcgtc ccgagacgcg agttatgtgt ttgcaaagcg tgccctaaaa    1080
   tctgcaaact atgcagagat ggcatttaat gtatgcggct tgatcctttc tgctgagaaa    1140
   tctttcgcca atagagtaga cgagaataaa cagctgctta aacagatcca agaaagtgta    1200
   gagtcatttc gggacatata caagagattc tctgagtatc agaaggaaca gaactcactg    1260
   ctgatgtcta acctatctac ccttcatatt atcacagaca gaggtggcaa gactgacaat    1320
   ccagattctc ccacaaggtc cccctctgtt tttgcaaaaa caaaagagaa caagaccaaa    1380
   gcaactaggt ttgacccgtc tatggagacc atgggggata tgaggtataa accagaccta    1440
   ctccgagagg atgaatttag agaagagatc cgcaacccgg tgtaccagga aagagatacc    1500
   gaacccagag catccaatgc atcacgtctg cttccatcaa gagagaagcc tacaatacac    1560
   tccctcaagc tcgtcataga gagcagtccc ctaagcagag ctgaaaaggc agcatatgtg    1620
   aaatcattat ccaagtgcaa gacagaccaa gaagttaagg ccgtcatgga gcttgtagaa    1680
   gaagacatag aatcactgac taattaa                                       1707
<210> 4
   <211> 568
   <212> PRT
   <213> Sendai Virus
<400> 4
   Met Asp Gln Asp Ala Leu Ile Ser Lys Glu Asp Ser Glu Val Glu Arg
   1               5                   10                  15
   Glu Ala Ser Gly Gly Arg Glu Ser Leu Ser Asp Val Ile Gly Phe Leu
               20                  25                  30
   Asp Ala Val Leu Ser Ser Glu Pro Thr Asp Ile Gly Gly Asp Arg Ser
           35                  40                  45
   Trp Leu His Asn Thr Ile Asn Thr Leu Gln Arg Pro Gly Ser Thr His
       50                  55                  60
   Arg Val Lys Gly Glu Gly Glu Gly Glu Val Ser Thr Ser Ser Thr Gln
   65                  70                  75                  80
   Asp Asn Arg Ser Gly Glu Glu Ser Arg Val Ser Gly Gly Thr Ser Glu
                   85                  90                  95
   Pro Glu Ala Glu Ala His Ala Arg Asn Val Asp Lys Gln Asn Ile His
               100                 105                 110
   Trp Ala Thr Gly Arg Gly Ala Ser Thr Asp Ser Val Pro Gln Asp Leu
           115                 120                 125
   Gly Asn Gly Arg Asp Ser Gly Ile Leu Glu Asp Pro Pro Asn Glu Gly
       130                 135                 140
   Gly Tyr Pro Arg Ser Gly Ala Glu Asp Glu Asn Arg Glu Met Ala Ala
   145                 150                 155                 160
   Asn Pro Asp Lys Arg Gly Glu Asp Gln Ala Glu Gly Leu Pro Glu Glu
                   165                 170                 175
   Ile Arg Arg Ser Ala Pro Leu Pro Asp Glu Arg Glu Gly Arg Ala Asp
               180                 185                 190
   Asn Asn Gly Arg Gly Val Glu Pro Gly Ser Pro His Ser Ala Arg Val
           195                 200                 205
   Thr Gly Val Leu Val Ile Pro Ser Pro Glu Leu Glu Glu Ala Val Leu
       210                 215                 220
   Gln Arg Asn Lys Arg Arg Pro Ala Asn Ser Gly Ser Arg Ser Leu Thr
   225                 230                 235                 240
   Pro Val Val Val Pro Ser Thr Arg Ser Pro Pro Pro Asp His Asp Asn
                   245                 250                 255
   Ser Thr Arg Ser Pro Pro Arg Lys Pro Pro Thr Thr Gln Asp Glu His
               260                 265                 270
   Thr Asn Pro Arg Asn Thr Pro Ala Val Arg Ile Lys Asp Arg Arg Pro
           275                 280                 285
   Pro Thr Gly Thr Arg Ser Ala Pro Asp Arg Pro Thr Asp Gly Tyr Pro
       290                 295                 300
   Thr His Pro Ser Pro Glu Thr Asp Ala Thr Lys Lys Gly Ile Glu Glu
   305                 310                 315                 320
   Asn Thr Ser Ser Met Lys Glu Met Ala Thr Leu Leu Thr Ser Leu Gly
                   325                 330                 335
   Val Ile Gln Ser Ala Gln Glu Phe Glu Ser Ser Arg Asp Ala Ser Tyr
               340                 345                 350
   Val Phe Ala Lys Arg Ala Leu Lys Ser Ala Asn Tyr Ala Glu Met Ala
   355 360 365
   Phe Asn Val Cys Gly Leu Ile Leu Ser Ala Glu Lys Ser Phe Ala Asn
   370 375 380
   Arg Val Asp Glu Asn Lys Gln Leu Leu Lys Gln Ile Gln Glu Ser Val
   385 390 395 400
   Glu Ser Phe Arg Asp Ile Tyr Lys Arg Phe Ser Glu Tyr Gln Lys Glu
   405 410 415
   Gln Asn Ser Leu Leu Met Ser Asn Leu Ser Thr Leu His Ile Ile Thr
   420 425 430
   Asp Arg Gly Gly Lys Thr Asp Asn Pro Asp Ser Pro Thr Arg Ser Pro
   435 440 445
   Ser Val Phe Ala Lys Thr Lys Glu Asn Lys Thr Lys Ala Thr Arg Phe
   450 455 460
   Asp Pro Ser Met Glu Thr Met Gly Asp Met Arg Tyr Lys Pro Asp Leu
   465 470 475 480
   Leu Arg Glu Asp Glu Phe Arg Glu Glu Ile Arg Asn Pro Val Tyr Gln
   485 490 495
   Glu Arg Asp Thr Glu Pro Arg Ala Ser Asn Ala Ser Arg Leu Leu Pro
   500 505 510
   Ser Arg Glu Lys Pro Thr Ile His Ser Leu Lys Leu Val Ile Glu Ser
   515 520 525
   Ser Pro Leu Ser Arg Ala Glu Lys Ala Ala Tyr Val Lys Ser Leu Ser
   530 535 540
   Lys Cys Lys Thr Asp Gln Glu Val Lys Ala Val Met Glu Leu Val Glu
   545 550 555 560
   Glu Asp Ile Glu Ser Leu Thr Asn
   565
<210> 5
   <211> 5
   <212> PRT
   <213> Sendai Virus
<400> 5
   Val Pro Gln Ser Arg
   1 5
<210> 6
   <211> 5
   <212> PRT
   <213> Newcastle Disease Virus
<400> 6
   Arg Arg Gln Lys Arg
   1 5
<210> 7
   <211> 348
   <212> DNA/RNA
   <213> Sendai Virus
<400> 7
   acggcttcgg ctacacttcc cgcatggatc aagatgccct catttctaaa gaagattctg 60
   aagttgagag ggaggcgtca ggaggacgag agtcgctctc ggatgttatc ggattcctcg 120
   acgcagtcct atcaagtgaa ccaactgaca tcggagggga cagaagctgg ctccacaata 180
   ccatcaacac cctccaaagg ccaggctcta cccacagagt caaaggtgag ggcgagggag 240
   aagtctcgac atcgtcgacc caagataatc gatcaggtga ggagagtaga gtctctgggg 300
   gaacaagcga gccagaggca gaagcacatg ctagaaacgt tgataaac 348
<210> 8
   <211> 215
   <212> PRT
   <213> Sendai Virus
<400> 8
   Thr Ala Ser Ala Thr Leu Pro Ala Trp Ile Lys Met Pro Ser Phe Leu
   1               5                   10                  15
   Lys Lys Ile Leu Lys Leu Arg Gly Arg Arg Gln Glu Asp Glu Ser Arg
               20                  25                  30
   Ser Arg Met Leu Ser Asp Ser Ser Thr Gln Ser Tyr Gln Val Asn Gln
           35                  40                  45
   Leu Thr Ser Glu Gly Thr Glu Ala Gly Ser Thr Ile Pro Ser Thr Pro
       50                  55                  60
   Ser Lys Gly Gln Ala Leu Pro Thr Glu Ser Lys Val Arg Ala Arg Glu
   65                  70                  75                  80
   Lys Ser Arg His Arg Arg Pro Lys Ile Ile Asp Gln Val Arg Arg Val
                   85                  90                  95
   Glu Ser Leu Gly Glu Gln Ala Ser Gln Arg Gln Lys His Met Leu Glu
               100                 105                 110
   Thr Leu Ile Asn Lys Ile Tyr Thr Gly Pro Leu Gly Glu Glu Leu Val
           115                 120                 125
   Gln Thr Leu Tyr Leu Arg Ile Trp Ala Met Glu Glu Thr Pro Glu Ser
       130                 135                 140
   Leu Lys Ile Leu Gln Met Arg Glu Asp Ile Arg Asp Gln Val Leu Lys
   145                 150                 155                 160
   Met Lys Thr Glu Arg Trp Leu Arg Thr Leu Ile Arg Gly Glu Lys Thr
                   165                 170                 175
   Lys Leu Lys Asp Phe Gln Lys Arg Tyr Glu Glu Val His Pro Tyr Leu
               180                 185                 190
   Met Lys Glu Lys Val Glu Gln Ile Ile Met Glu Glu Ala Trp Ser Leu
           195                 200                 205
   Ala Ala His Ile Val Gln Glu
       210                 215
<210> 9
   <211> 615
   <212> DNA/RNA
   <213> Sendai Virus
<400> 9
   atgccctcat ttctaaagaa gattctgaag ttgagaggga ggcgtcagga ggacgagagt     60
   cgctctcgga tgttatcgga ttcctcgacg cagtcctatc aagtgaacca actgacatcg    120
   gaggggacag aagctggctc cacaatacca tcaacaccct ccaaaggcca ggctctaccc    180
   acagagtcaa aggtgagggc gagggagaag tctcgacatc gtcgacccaa gataatcgat    240
   caggtgagga gagtagagtc tctgggggaa caagcgagcc agaggcagaa gcacatgcta    300
   gaaacgttga taaacaaaat atacactggg ccactgggag aggagctagt acagactctg    360
   tacctcagga tctgggcaat ggaagagact ccggaatcct tgaagatcct ccaaatgagg    420
   gaggatatcc gagatcaggt gctgaagatg aaaaccgaga gatggctgcg aaccctgata    480
   agaggggaga agaccaagct gaaggacttc cagaagagat acgaagaagt gcacccctac    540
   ctgatgaaag agaaggtaga gcagataata atggaagagg cgtggagcct ggcagcccac    600
   atagtgcaag agtga                                                     615
<210> 10
   <211> 204
   <212> PRT
   <213> Sendai Virus
<400> 10
   Met Pro Ser Phe Leu Lys Lys Ile Leu Lys Leu Arg Gly Arg Arg Gln
   1               5                   10                  15
   Glu Asp Glu Ser Arg Ser Arg Met Leu Ser Asp Ser Ser Thr Gln Ser
               20                  25                  30
   Tyr Gln Val Asn Gln Leu Thr Ser Glu Gly Thr Glu Ala Gly Ser Thr
           35                  40                  45
   Ile Pro Ser Thr Pro Ser Lys Gly Gln Ala Leu Pro Thr Glu Ser Lys
       50                  55                  60
   Val Arg Ala Arg Glu Lys Ser Arg His Arg Arg Pro Lys Ile Ile Asp
   65                  70                  75                  80
   Gln Val Arg Arg Val Glu Ser Leu Gly Glu Gln Ala Ser Gln Arg Gln
                   85                  90                  95
   Lys His Met Leu Glu Thr Leu Ile Asn Lys Ile Tyr Thr Gly Pro Leu
               100                 105                 110
   Gly Glu Glu Leu Val Gln Thr Leu Tyr Leu Arg Ile Trp Ala Met Glu
           115                 120                 125
   Glu Thr Pro Glu Ser Leu Lys Ile Leu Gln Met Arg Glu Asp Ile Arg
       130                 135                 140
   Asp Gln Val Leu Lys Met Lys Thr Glu Arg Trp Leu Arg Thr Leu Ile
   145                 150                 155                 160
   Arg Gly Glu Lys Thr Lys Leu Lys Asp Phe Gln Lys Arg Tyr Glu Glu
                   165                 170                 175
   Val His Pro Tyr Leu Met Lys Glu Lys Val Glu Gln Ile Ile Met Glu
               180                 185                 190
   Glu Ala Trp Ser Leu Ala Ala His Ile Val Gln Glu
           195                 200
<210> 11
   <211> 1108
   <212> DNA
   <213> Sendai Virus
<400> 11
   tggatcaaga tgccctcatt tctaaagaag attctgaagt tgagagggag gcgtcaggag      60
   gacgagagtc gctctcggat gttatcggat tcctcgacgc agtcctatca agtgaaccaa     120
   ctgacatcgg aggggacaga agctggctcc acaataccat caacaccctc caaaggccag     180
   gctctaccca cagagtcaaa ggtgagggcg agggagaagt ctcgacatcg tcgacccaag     240
   ataatcgatc aggtgaggag agtagagtct ctgggggaac aagcgagcca gaggcagaag     300
   cacatgctag aaacgttgat aaacaaaata tacactgggc cactgggaga ggagctagta     360
   cagactctgt acctcaggat ctgggcaatg gaagagactc cggaatcctt gaagatcctc     420
   caaatgaggg aggatatccg agatcaggtg ctgaagatga aaaccgagag atggctgcga     480
   accctgataa gaggggagaa gaccaagctg aaggacttcc agaagagata cgaagaagtg     540
   cacccctacc tgatgaaaga gaaggtagag cagataataa tggaagaggc gtggagcctg     600
   gcagcccaca tagtgcaaga gtgactggag tcttggtgat ccccagtcct gagctcgaag     660
   aggccgtgct acaaaggaac aagagacgac ccgccaacag cgggtccaga tctctcaccc     720
   cagtggtcgt gcctagcact cggtctccac caccggacca tgacaatagt acaaggtcac     780
   caccaaggaa acccccaacc acgcaggatg agcacaccaa ccccaggaac acccccgccg     840
   tcaggatcaa ggatcggaga cccccaacag ggactcgctc cgccccagac cgcccgaccg     900
   acggctatcc aacccaccca agtccagaga ccgatgcaac aaaaaagggc atagaagaga     960
   acacatcatc tatgaaagag atggctacat tgttaacgag tcttggtgta atccagtctg    1020
   ctcaagaatt cgagtcgtcc cgagacgcga gttatgtgtt tgcaaagcgt gccctaaaat    1080
   ctgcaaacta tgcagagatg gcatttaa                                       1108
<210> 12
   <211> 369
   <212> PRT
   <213> Sendai Virus
<400> 12
   Met Asp Gln Asp Ala Leu Ile Ser Lys Glu Asp Ser Glu Val Glu Arg
   1               5                   10                  15
   Glu Ala Ser Gly Gly Arg Glu Ser Leu Ser Asp Val Ile Gly Phe Leu
               20                  25                  30
   Asp Ala Val Leu Ser Ser Glu Pro Thr Asp Ile Gly Gly Asp Arg Ser
           35                  40                  45
   Trp Leu His Asn Thr Ile Asn Thr Leu Gln Arg Pro Gly Ser Thr His
       50                  55                  60
   Arg Ala Lys Gly Glu Gly Glu Gly Glu Val Ser Thr Ser Ser Thr Gln
   65                  70                  75                  80
   Asp Asn Arg Ser Gly Glu Glu Ser Arg Val Ser Gly Gly Thr Ser Glu
                   85                  90                  95
   Pro Glu Ala Glu Ala His Ala Arg Asn Val Asp Lys Gln Asn Ile His
               100                 105                 110
   Trp Ala Thr Gly Arg Gly Ala Ser Thr Asp Ser Val Pro Gln Asp Leu
           115                 120                 125
   Gly Asn Gly Arg Asp Ser Gly Ile Leu Glu Asp Pro Pro Asn Glu Gly
       130                 135                 140
   Gly Tyr Pro Arg Ser Gly Ala Glu Asp Glu Asn Arg Glu Met Ala Ala
   145                 150                 155                 160
   Asn Pro Asp Lys Arg Gly Glu Asp Gln Ala Glu Gly Leu Pro Glu Glu
                   165                 170                 175
   Ile Arg Arg Ser Ala Pro Leu Pro Asp Glu Gly Glu Gly Arg Ala Asp
               180                 185                 190
   Asn Asn Gly Arg Gly Val Glu Ser Gly Ser Pro His Ser Ala Arg Val
           195                 200                 205
   Thr Gly Val Leu Val Ile Pro Ser Pro Glu Leu Glu Glu Ala Val Leu
       210                 215                 220
   Gln Arg Asn Lys Arg Arg Pro Ala Asn Ser Gly Ser Arg Ser Leu Thr
   225                 230                 235                 240
   Pro Val Val Val Pro Ser Thr Arg Ser Pro Pro Pro Asp His Asp Asn
                   245                 250                 255
   Ser Thr Arg Ser Pro Pro Arg Lys Pro Pro Thr Thr Gln Asp Glu His
               260                 265                 270
   Thr Asn Pro Arg Asn Thr Pro Ala Val Arg Ile Lys Asp Arg Arg Pro
           275                 280                 285
   Pro Thr Gly Thr Arg Ser Ala Pro Asp Arg Pro Thr Asp Gly Tyr Pro
       290                 295                 300
   Thr His Pro Gly Pro Glu Thr Asp Ala Thr Lys Lys Gly His Arg Arg
   305                 310                 315                 320
   Glu His Ile Ile Tyr Glu Arg Asp Gly Tyr Ile Val Asn Glu Ser Trp
                   325                 330                 335
   Cys Asn Pro Val Cys Ser Arg Ile Arg Val Ile Ser Arg Arg Glu Leu
               340                 345                 350
   Cys Val Cys Lys Ala Cys Pro Lys Ile Cys Lys Leu Cys Arg Asp Asp
   355 360 365
   Ile
<210> 13
   <211> 955
   <212> DNA/RNA
   <213> Sendai Virus
<400> 13
   atggatcaag atgccttcat tcttaaagaa gattctgaag ttgagaggga ggcgccagga     60
   ggaagagagt cgctctcgga tgttatcgga ttcctcgatg ctgtcctgtc gagtgaacca    120
   actgacatcg gaggggacag gagctggctc cacaacacca tcaacactcc ccaaggacca    180
   ggctctgccc atagagccaa aagtgagggc gaaggagaag tctcaacacc gtcgacccaa    240
   gataatcgat caggtgagga gagtagagtc tctgggagaa caagcaagcc agaggcagaa    300
   gcacatgctg gaaaccttga taaacaaaat atacaccggg cctttggggg aagaactggt    360
   acaaactctg tatctcagga tctgggcgat ggaggagact ccggaatcct tgaaaatcct    420
   ccaaatgaga gaggatatcc gagatcaggt attgaagatg aaaacagaga gatggctgcg    480
   caccctgata agaggggaga agaccaagct gaaggacttc cagaagaggt acgaggaggt    540
   acatccctac ctgatgaagg agaaggtgga gcaagtaata atggaagaag catggagcct    600
   ggcagctcac atagtgcaag agtaactggg gtcctggtga ttcctagccc cgaactcgaa    660
   gaggctgtgc tacggaggaa caaaagaaga cctaccaaca gtgggtccaa acctcttact    720
   ccagcaaccg tgcctggcac ccggtcccca ccgctgaatc gttacaacag cacagggtca    780
   ccaccaggaa aacccccatc tacacaggat gagcacatca actctgggga cacccccgcc    840
   gtcagggtca aagaccggaa accaccaata gggacccgct ctgtctcaga ttgtccagcc    900
   aacggccgcc caatccaccc gggtctagag accgactcaa caaaaaaggg catag         955
<210> 14
   <211> 318
   <212> PRT
   <213> Sendai Virus
<400> 14
   Met Asp Gln Asp Ala Phe Ile Leu Lys Glu Asp Ser Glu Val Glu Arg
   1 5 10 15
   Glu Ala Pro Gly Gly Arg Glu Ser Leu Ser Asp Val Ile Gly Phe Leu
   20 25 30
   Asp Ala Val Leu Ser Ser Glu Pro Thr Asp Ile Gly Gly Asp Arg Ser
           35                  40                  45
   Trp Leu His Asn Thr Ile Asn Thr Pro Gln Gly Pro Gly Ser Ala His
       50                  55                  60
   Arg Ala Lys Ser Glu Gly Glu Gly Glu Val Ser Thr Pro Ser Thr Gln
   65                  70                  75                  80
   Asp Asn Arg Ser Gly Glu Glu Ser Arg Val Ser Gly Arg Thr Ser Lys
                   85                  90                  95
   Pro Glu Ala Glu Ala His Ala Gly Asn Leu Asp Lys Gln Asn Ile His
               100                 105                 110
   Arg Ala Phe Gly Gly Arg Thr Gly Thr Asn Ser Val Ser Gln Asp Leu
           115                 120                 125
   Gly Asp Gly Gly Asp Ser Gly Ile Leu Glu Asn Pro Pro Asn Glu Arg
       130                 135                 140
   Gly Tyr Pro Arg Ser Gly Ile Glu Asp Glu Asn Arg Glu Met Ala Ala
   145                 150                 155                 160
   His Pro Asp Lys Arg Gly Glu Asp Gln Ala Glu Gly Leu Pro Glu Glu
                   165                 170                 175
   Val Arg Gly Gly Thr Ser Leu Pro Asp Glu Gly Glu Gly Gly Ala Ser
               180                 185                 190
   Asn Asn Gly Arg Ser Met Glu Pro Gly Ser Ser His Ser Ala Arg Val
           195                 200                 205
   Thr Gly Val Leu Val Ile Pro Ser Pro Glu Leu Glu Glu Ala Val Leu
       210                 215                 220
   Arg Arg Asn Lys Arg Arg Pro Thr Asn Ser Gly Ser Lys Pro Leu Thr
   225                 230                 235                 240
   Pro Ala Thr Val Pro Gly Thr Arg Ser Pro Pro Leu Asn Arg Tyr Asn
                   245                 250                 255
   Ser Thr Gly Ser Pro Pro Gly Lys Pro Pro Ser Thr Gln Asp Glu His
               260                 265                 270
   Ile Asn Ser Gly Asp Thr Pro Ala Val Arg Val Lys Asp Arg Lys Pro
           275                 280                 285
   Pro Ile Gly Thr Arg Ser Val Ser Asp Cys Pro Ala Asn Gly Arg Pro
   290 295 300
   Ile His Pro Gly Leu Glu Thr Asp Ser Thr Lys Lys Gly Ile
   305 310 315
<210> 15
   <211> 546
   <212> DNA/RNA
   <213> Sendai Virus
<400> 15
   atgttatcgg attcctcgac gcagtcctat caagtgaacc aactgacatc ggaggggaca     60
   gaagctggct ccacaatacc atcaacaccc tccaaaggcc aggctctacc cacagagtca    120
   aaggtgaggg cgagggagaa gtctcgacat cgtcgaccca agataatcga tcaggtgagg    180
   agagtagagt ctctggggga acaagcgagc cagaggcaga agcacatgct agaaacgttg    240
   ataaacaaaa tatacactgg gccactggga gaggagctag tacagactct gtacctcagg    300
   atctgggcaa tggaagagac tccggaatcc ttgaagatcc tccaaatgag ggaggatatc    360
   cgagatcagg tgctgaagat gaaaaccgag agatggctgc gaaccctgat aagaggggag    420
   aagaccaagc tgaaggactt ccagaagaga tacgaagaag tgcaccccta cctgatgaaa    480
   gagaaggtag agcagataat aatggaagag gcgtggagcc tggcagccca catagtgcaa    540
   gagtga                                                               546
<210> 16
   <211> 181
   <212> PRT
   <213> Sendai Virus
<400> 16
   Met Leu Ser Asp Ser Ser Thr Gln Ser Tyr Gln Val Asn Gln Leu Thr
   1               5                   10                  15
   Ser Glu Gly Thr Glu Ala Gly Ser Thr Ile Pro Ser Thr Pro Ser Lys
               20                  25                  30
   Gly Gln Ala Leu Pro Thr Glu Ser Lys Val Arg Ala Arg Glu Lys Ser
           35                  40                  45
   Arg His Arg Arg Pro Lys Ile Ile Asp Gln Val Arg Arg Val Glu Ser
       50                  55                  60
   Leu Gly Glu Gln Ala Ser Gln Arg Gln Lys His Met Leu Glu Thr Leu
   65                  70                  75                  80
   Ile Asn Lys Ile Tyr Thr Gly Pro Leu Gly Glu Glu Leu Val Gln Thr
                   85                  90                  95
   Leu Tyr Leu Arg Ile Trp Ala Met Glu Glu Thr Pro Glu Ser Leu Lys
               100                 105                 110
   Ile Leu Gln Met Arg Glu Asp Ile Arg Asp Gln Val Leu Lys Met Lys
           115                 120                 125
   Thr Glu Arg Trp Leu Arg Thr Leu Ile Arg Gly Glu Lys Thr Lys Leu
       130                 135                 140
   Lys Asp Phe Gln Lys Arg Tyr Glu Glu Val His Pro Tyr Leu Met Lys
   145                 150                 155                 160
   Glu Lys Val Glu Gln Ile Ile Met Glu Glu Ala Trp Ser Leu Ala Ala
                   165                 170                 175
   His Ile Val Gln Glu
               180
<210> 17
   <211> 528
   <212> PRT
   <213> Sendai Virus
<400> 17
   Ala Thr Gly Cys Thr Gly Thr Cys Cys Thr Gly Thr Cys Gly Ala Gly
   1               5                   10                  15
   Thr Gly Ala Ala Cys Cys Ala Ala Cys Thr Gly Ala Cys Ala Thr Cys
               20                  25                  30
   Gly Gly Ala Gly Gly Gly Gly Ala Cys Ala Gly Ala Ala Gly Cys Thr
           35                  40                  45
   Gly Gly Cys Thr Cys Cys Ala Cys Ala Ala Cys Ala Cys Cys Ala Thr
       50                  55                  60
   Cys Ala Ala Cys Ala Cys Thr Cys Cys Cys Cys Ala Ala Gly Gly Ala
   65                  70                  75                  80
   Cys Cys Ala Gly Gly Cys Thr Cys Thr Gly Cys Cys Cys Ala Thr Ala
                   85                  90                  95
   Gly Ala Gly Cys Cys Ala Ala Ala Ala Gly Thr Gly Ala Gly Gly Gly
               100                 105                 110
   Cys Gly Ala Ala Gly Gly Ala Gly Ala Ala Gly Thr Cys Thr Cys Ala
           115                 120                 125
   Ala Cys Ala Cys Cys Gly Thr Cys Gly Ala Cys Cys Cys Ala Ala Gly
       130                 135                 140
   Ala Thr Ala Ala Thr Cys Gly Ala Thr Cys Ala Gly Gly Thr Gly Ala
   145                 150                 155                 160
   Gly Gly Ala Gly Ala Gly Thr Ala Gly Ala Gly Thr Cys Thr Cys Thr
                   165                 170                 175
   Gly Gly Gly Ala Gly Ala Ala Cys Ala Ala Gly Cys Ala Ala Gly Cys
               180                 185                 190
   Cys Ala Gly Ala Gly Gly Cys Ala Gly Ala Ala Gly Cys Ala Cys Ala
           195                 200                 205
   Thr Gly Cys Thr Gly Gly Ala Ala Ala Cys Cys Thr Thr Gly Ala Thr
       210                 215                 220
   Ala Ala Ala Cys Ala Ala Ala Ala Thr Ala Thr Ala Cys Ala Cys Cys
   225                 230                 235                 240
   Gly Gly Gly Cys Cys Thr Thr Thr Gly Gly Gly Gly Gly Ala Ala Gly
                   245                 250                 255
   Ala Ala Cys Thr Gly Gly Thr Ala Cys Ala Ala Ala Cys Thr Cys Thr
               260                 265                 270
   Gly Thr Ala Thr Cys Thr Cys Ala Gly Gly Ala Thr Cys Thr Gly Gly
           275                 280                 285
   Gly Cys Gly Ala Thr Gly Gly Ala Gly Gly Ala Gly Ala Cys Thr Cys
       290                 295                 300
   Cys Gly Gly Ala Ala Thr Cys Cys Thr Thr Gly Ala Ala Ala Ala Thr
   305                 310                 315                 320
   Cys Cys Thr Cys Cys Ala Ala Ala Thr Gly Ala Gly Ala Gly Ala Gly
                   325                 330                 335
   Gly Ala Thr Ala Thr Cys Cys Gly Ala Gly Ala Thr Cys Ala Gly Gly
               340                 345                 350
   Thr Ala Thr Thr Gly Ala Ala Gly Ala Thr Gly Ala Ala Ala Ala Cys
           355                 360                 365
   Ala Gly Ala Gly Ala Gly Ala Thr Gly Gly Cys Thr Gly Cys Gly Cys
       370                 375                 380
   Ala Cys Cys Cys Thr Gly Ala Thr Ala Ala Gly Ala Gly Gly Gly Gly
   385                 390                 395                 400
   Ala Gly Ala Ala Gly Ala Cys Cys Ala Ala Gly Cys Thr Gly Ala Ala
                   405                 410                 415
   Gly Gly Ala Cys Thr Thr Cys Cys Ala Gly Ala Ala Gly Ala Gly Gly
               420                 425                 430
   Thr Ala Cys Gly Ala Gly Gly Ala Gly Gly Thr Ala Cys Ala Thr Cys
           435                 440                 445
   Cys Cys Thr Ala Cys Cys Thr Gly Ala Thr Gly Ala Ala Gly Gly Ala
       450                 455                 460
   Gly Ala Ala Gly Gly Thr Gly Gly Ala Gly Cys Ala Ala Gly Thr Ala
   465                 470                 475                 480
   Ala Thr Ala Ala Thr Gly Gly Ala Ala Gly Ala Ala Gly Cys Ala Thr
                   485                 490                 495
   Gly Gly Ala Gly Cys Cys Thr Gly Gly Cys Ala Gly Cys Thr Cys Ala
               500                 505                 510
   Cys Ala Thr Ala Gly Thr Gly Cys Ala Ala Gly Ala Gly Thr Ala Ala
           515                 520                 525
<210> 18
   <211> 175
   <212> PRT
   <213> Sendai Virus
<400> 18
   Met Leu Ser Cys Arg Val Asn Gln Leu Thr Ser Glu Gly Thr Glu Ala
   1               5                   10                  15
   Gly Ser Thr Thr Pro Ser Thr Leu Pro Lys Asp Gln Ala Leu Pro Ile
               20                  25                  30
   Glu Pro Lys Val Arg Ala Lys Glu Lys Ser Gln His Arg Arg Pro Lys
           35                  40                  45
   Ile Ile Asp Gln Val Arg Arg Val Glu Ser Leu Gly Glu Gln Ala Ser
       50                  55                  60
   Gln Arg Gln Lys His Met Leu Glu Thr Leu Ile Asn Lys Ile Tyr Thr
   65                  70                  75                  80
   Gly Pro Leu Gly Glu Glu Leu Val Gln Thr Leu Tyr Leu Arg Ile Trp
                   85                  90                  95
   Ala Met Glu Glu Thr Pro Glu Ser Leu Lys Ile Leu Gln Met Arg Glu
               100                 105                 110
   Asp Ile Arg Asp Gln Val Leu Lys Met Lys Thr Glu Arg Trp Leu Arg
           115                 120                 125
   Thr Leu Ile Arg Gly Glu Lys Thr Lys Leu Lys Asp Phe Gln Lys Arg
       130                 135                 140
   Tyr Glu Glu Val His Pro Tyr Leu Met Lys Glu Lys Val Glu Gln Val
   145                 150                 155                 160
   Ile Met Glu Glu Ala Trp Ser Leu Ala Ala His Ile Val Gln Glu
                   165                 170                 175
<210> 19
   <211> 1730
   <212> DNA/RNA
   <213> künstliche Sequenz
<220> keine
   <223> Klonierungsbereich von pSL1180
<400> 19
   acggcttcgg ctacacttac cgcatggatc aagatgcctt cattcttaaa gaagattctg     60
   aagttgagag ggaggcgcca ggaggaagag agtcgctctc ggatgttatc ggattcctcg    120
   atgctgtcct gtcgagtgaa ccaactgaca tcggagggga cagaagctgg ctccacaaca    180
   ccatcaacac tccccaagga ccaggctctg cccatagagc caaaagtgag ggcgaaggag    240
   aagtctcaac accgtcgacc caagataatc gatcaggtga ggagagtaga gtctctggga    300
   gaacaagcaa gccagaggca gaagcacatg ctggaaacct tgataaacaa aatatacacc    360
   gggcctttgg gggaagaact ggtacaaact ctgtatctca ggatctgggc gatggaggag    420
   actccggaat ccttgaaaat cctccaaatg agagaggata tccgagatca ggtattgaag    480
   atgaaaacag agagatggct gcgcaccctg ataagagggg agaagaccaa gctgaaggac    540
   ttccagaaga ggtacgagga ggtacatccc tacctgatga aggagaaggt ggagcaagta    600
   ataatggaag aagcatggag cctggcagct cacatagtgc aagagtaact ggggtcctgg    660
   tgattcctag ccccgaactc gaagaggctg tgctacggag gaacaaaaga agacctacca    720
   acagtgggtc caaacctctt actccagcaa ccgtgcctgg cacccggtcc ccaccgctga    780
   atcgttacaa cagcacaggg tcaccaccag gaaaaccccc atctacacag gatgagcaca    840
   tcaactctgg ggacaccccc gccgtcaggg tcaaagaccg gaaaccacca atagggaccc    900
   gctctgtctc agattgtcca gccaacggcc gcccaatcca cccgggtcta gagaccgact    960
   caacaaaaaa gggcatagga gagaacacat catctatgaa agagatggct acattgttga   1020
   cgagtcttgg tgtaatccag tctgctcaag aattcgagtc atcccgagac gcgagttatg   1080
   tgtttgcaag acgtgcccta aagtctgcaa actatgcaga gatgacattc aatgtatgcg   1140
   gcctgatcct ttctgccgag aaatcttccg ctcgtaaggt agatgagaac aaacaactgc   1200
   tcaaacagat ccaagagagc gtggaatcat tccgggatat ttacaagaga ttctctgagt   1260
   atcagaaaga acagaactca ttgctgatgt ccaacctatc tacacttcat atcatcacag   1320
   atagaggtgg caagactgac aacacagact cccttacaag gtccccctcc gtttttgcaa   1380
   aatcaaaaga gaacaagact aaggctacca ggtttgaccc atctatggag accctagaag   1440
   atatgaagta caaaccggac ctaatccgag aggatgaatt tagagatgag atccgcaacc   1500
   cggtgtacca agagagggac acagaaccca gggcctcaaa cgcatcacgc ctcctcccct   1560
   ccaaagagaa gcccacaatg cactctctca ggctcgtcat agagagcagt cccctaagca   1620
   gagctgagaa agcagcatat gtgaaatcat tatccaagtg caagacagac caagaggtta   1680
   aggcagtcat ggaactcgta gaagaggaca tagagtcact gaccaactag               1730
<210> 20
   <211> 51
   <212> DNA/RNA
   <213> künstliche Sequenz
<220> keine
   <223> PCR-Primer 1
<400> 20
   cgcatggatc aagacgcctt cattctaaaa gaagattctg aagtagagag g 51
<210> 21
   <211> 51
   <212> DNA/RNA
   <213> künstliche Sequenz
<220> keine
   <223> P-Gen
<400> 21
   cgcatggatc aagatgcctt cattcttaaa gaagattctg aagttgagag g 51
<210> 22
   <211> 51
   <212> DNA/RNA
   <213> künstliche Sequenz
<220> keine
   <223> P-Gen mut.
<400> 22
   cgcatggatc aagacgcctt cattctaaaa gaagattctg aagtagagag g 51
<210> 23
   <211> 38
   <212> DNA/RNA
   <213> künstliche Sequenz
<220> keine
   <223> C-Gen mut.
<400> 23
   acgccttcat tctaaaagaa gattctgaag tagagagg 38
<210> 24
   <211> 36
   <212> DNA/RNA
   <213> künstliche Sequenz
<220> keine
   <223> PCR-Primer 2
<400> 24
   ctctcggacg ttatcggatt cctcgacgct gtcctg 36
<210> 25
   <211> 36
   <212> DNA/RNA
   <213> künstliche Sequenz
<220> keine
   <223> P-Gen
<400> 25
   ctctcggacg ttatcggatt cctcgacgct gtcctg 36
<210> 26
   <211> 36
   <212> DNA/RNA
   <213> künstliche Sequenz
<220> keine
   <223> Y1-Gen
<400> 26
   ctctcggatg ttatcggatt cctcgatgct gtcctg 36
<210> 27
   <211> 36
   <212> DNA/RNA
   <213> künstliche Sequenz
<220> keine
   <223> Y1-Gen mut.
<400> 27
   ctctcggacg ttatcggatt cctcgacgct gtcctg 36
<210> 28
   <211> 42
   <212> DNA/RNA
   <213> künstliche Sequenz
<220> keine
   <223> PCR-Primer 3
<400> 28
   gactcaacaa agaaaggcat aggtgagaac acatcatcta tg 42
<210> 29
   <211> 42
   <212> DNA/RNA
   <213> künstliche Sequenz
<220> keine
   <223> P-Gen
<400> 29
   gactcaacaa aaaagggcat aggagagaac acatcatcta tg 42
<210> 30
   <211> 42
   <212> DNA/RNA
   <213> künstliche Sequenz
<220> keine
   <223> P-Gen mut.
<400> 30
   gactcaacaa agaaaggcat aggtgagaac acatcatcta tg 42
<210> 31
   <211> 43
   <212> DNA/RNA
   <213> künstliche Sequenz
<220> keine
   <223> V-Gen mut.
<400> 31
   gactcaacaa agaaagggca taggtgagaa cacatcatct atg 43
<210> 32
   <211> 44
   <212> DNA/RNA
   <213> künstliche Sequenz
<220> keine
   <223> W-Gen mut.
<400> 32
   gactcaacaa agaaaggggc ataggtgaga acacatcatc tatg 44
<210> 33
   <211> 22
   <212> DNA/RNA
   <213> künstliche Sequenz
<220> keine
   <223> Spaltstelle im F-Gen von SeV
<400> 33
   gttccacagtc gaga 22
<210> 34
   <211> 22
   <212> DNA/RNA
   <213> künstliche Sequenz
<220> keine
   <223> Spaltstelle im F-Gen von NDV
<400> 34
   cgtcgtcagaaga gg 22
2
1

## Beschreibung

Die vorliegende Erfindung betrifft ein genetisch modifiziertes Sendai Virus, eine dieses Sendai Virus aufweisende pharmazeutische Zusammensetzung, die Verwendung eines genetisch modifizierten Sendai Virus zur therapeutischen und/oder prophylaktischen Behandlung einer Tumorerkrankung und ein Verfahren zur Herstellung einer pharmazeutischen Zusammensetzung zur therapeutischen und/oder prophylaktischen Behandlung einer Tumorerkrankung.

Statistisch gesehen entwickelt jeder dritte Europäer im Laufe seines Lebens Krebs. In Deutschland erkranken etwa 395.000 Menschen jährlich an Krebs, davon rund 195.000 Frauen und 200.000 Männer. Die meisten Fälle treten im Alter von über 60 Jahren auf.

Solide Tumoren stellen nach wie vor große Herausforderungen an die klinische Onkologie dar. Eine wesentliche Verbesserung der Prognose einzelner Tumorerkrankungen kann nahezu ausschließlich durch die Einführung neuer Therapieprinzipien, integriert in multimodale Konzepte, erreicht werden.

Eines dieser neuen Therapieprinzipien betrifft den Einsatz von replizierenden Viren zur Behandlung von Tumoren. Dieser Ansatz wird als Virotherapie oder Onkolyse bezeichnet. Zahlreiche Viren besitzen onkolytische Eigenschaften mit einer präferenziellen Replikation in unterschiedlichen Tumorzellen im Vergleich zu einer reduzierten Replikation in gesunden Parenchymzellen. In mehreren klinischen Studien werden derzeit verschiedene Virotherapeutika getestet.

Von besonderem Interesse sind Viren der Familie *Paramyxoviridae.* Wichtige Mitglieder dieser Familie von behüllten Viren betreffen das zu der Gattung der Avulaviren zählende Newcastle-Disease-Virus, das zur Gattung der Morbilliviren zählende Masernvirus und das zur Gattung der Respiroviren zählende Sendai Virus.

Die Paramyxoviren weisen als Genom eine negative einzelsträngige RNA auf, d.h. ein RNA-Molekül, das Gene bzw. offene Leseraster ("Open Reading Frames"; ORFs) im Gegensinnmodus codiert. Bei dem Sendai Virus folgen auf eine 3'-Kopfregion des RNA-Genoms die viralen Gene N (Nucleocapsid), P (Phospho), M (Matrix), F (Fusion), HN (Hämagglutinin-Neuraminidase) und L (Large), gefolgt von der 5'-Schwanzregion.

Die N-, P- und L-Proteine sind für die Expression der von der genomischen RNA codierten Gene und für die autonome Replikation der RNA erforderlich. Das HN-Protein unterstützt die Infektion bestimmter Zelltypen. Das sogenannte Matrix-Protein (M) ist ein im Viruspartikel mit der Membran assoziiertes Strukturprotein.

Das F-Protein spielt eine zentrale Rolle bei der Infektion, indem es die Zellmembranfusion induziert, die erforderlich ist für die initiale Infektion und die Virusausbreitung auf benachbarte Zellen. Dieses wird als biologisch inaktive Vorstufe F0 in virusinfizierten Zellen synthetisiert und in der Lipidhülle des Virus, die von der Plasmamembran der Wirtszelle stammt, verankert. F0 wird durch eine sich im Respirationstrakt von Ratten und Mäusen befindliche und aus den Bronchialepithelzellen sezernierte Tryptase "Clara" in die aktiven Untereinheiten F1 und F2 gespalten. F1 und F2 sind in der Lage Zellmembranen zu fusionieren und damit die Infektion des Wirtes durch das Virus zu veranlassen. Die Spaltung von F0 stellt deshalb eine entscheidende Determinante für die Infektiosität und Pathogenität des Sendai Virus dar. Die Proteaserestriktion ist eine wichtige Determinante, durch die eine Infektion mit Sendai Virus in Mäusen auf den Respirationstrakt beschränkt bleibt und nicht zu einer systemischen Infektion führen kann.

Kinoh et al. (2004), Generation of a recombinant Sendai virus that is selectively activated and lyses human tumor cells expressing matrix metalloproteinses, Gene Ther. 11, S. 1137-1145, schlagen die Verwendung eines genetisch modifizierten Sendai Virus zur Behandlung von Tumorerkrankungen vor. Das Prinzip der genetischen Modifizierung besteht in der Einführung einer artifiziellen Spaltstelle in das virale F-Protein, die von tumorspezifischen Matrixmetalloproteinasen erkannt und gespalten werden kann und somit eine tumorspezifische Replikation der modifizierten Viren ermöglichen soll. Ferner weist das bekannte genetisch modifizierte Sendai Virus eine Deletion im viralen M-Protein auf, die zu einer Blockade der Freisetzung von Nachkommenviren führt, so dass eine Ausbreitung des Virus lediglich durch Zell-Zell-Kontakte per Fusion ermöglicht wird. Dieses modifizierten Sendai Virus wird auch in der EP 1 505 154 offenbart.

Kinoh et al. (2009), Generation of optimized and urokinase-targeted oncolytic Sendai virus vectors applicable for various human malignancies, Gene Ther. 16, S. 392-403, beschreiben ein genetisch modifiziertes Sendai Virus, bei dem eine Trunkierung von Aminosäuren im viralen F-Protein vorliegt, wodurch die Fusionsaktivität erhöht werden soll. Ferner weist das virale F-Protein eine sogenannte "Urokinase Type Plasminogen Activator (uPA) Sensitive Sequence" (SGRS) auf, durch die eine Spaltung und Aktivierung von F0 durch tumorspezifische Proteasen die Replikationsfähigkeit der Viren auf eine Vielzahl von Tumoren erweitert werden soll.

Elankumaran et al. (2010), Type I Interferone sensitive recombinant Newcastle-Disease-Virus for oncolytic virotherapy, Journal of Virology, Online-Veröffentlichung, schlagen die Verwendung von rekombinanten Newcastle-Disease-Viren (rNDV) zur Verwendung als Antitumoragens vor, die entweder eine Mutation im V-Protein aufweisen und als "rBC-Edit" bezeichnet werden, oder eine Mutation im F-Protein aufweisen und als "rLaSota V.F." bezeichnet werden.

In der US 2009/0175826 wird die Verwendung eines rekombinanten Newcastle-Disease-Virus (rNDV) als onkolytisches Agens vorgeschlagen, das ein Transgen aufweist, welches in Tumorzelllinien Apoptose induziert soll.

Die im Stand der Technik beschriebenen onkolytischen Viren haben sich jedoch bislang nicht bewährt. Eine klinische Anwendung mit einem definierten Wirkungsnachweis steht aus. Insbesondere weisen die bislang im Stand der Technik verwendeten onkolytischen Viren den Nachteil auf, dass sie in erhöhtem Maße auch Nicht-Tumorzellen infizieren und diese zerstören können. Zusätzlich ist unklar, bei welchen Tumorerkrankungen eine gute Wirkung einzelner Virussysteme erreicht werden kann. Dies macht die bislang verwendeten onkolytischen Viren für einen klinischen Einsatz unbrauchbar.

Vor diesem Hintergrund ist es die Aufgabe der vorliegenden Erfindung ein verbessertes onkolytisches Virus bereitzustellen, mit dem die Nachteile der bisher verwendeten onkolytischen Viren weitgehend vermieden werden. Insbesondere sollen solche onkolytischen Viren bereitgestellt werden, die in Tumorzellen replizieren und diese zerstören können, in Nicht-Tumorzellen jedoch stark eingeschränkt replizieren und damit einen ausreichenden epidemiologischen Sicherheitsaspekt erfüllen.

Diese Aufgabe wird durch die Bereitstellung eines genetisch modifizierten Sendai Virus (SeV), gelöst, das gegenüber dem Wildtyp (WT) in seinem F-Gen zumindest eine erste genetische Modifizierung und in seinem P-Gen zumindest eine zweite genetische Modifizierung aufweist, wobei durch die erste genetische Modifizierung die Codierungsnucleotidsequenz für die SeV-WT-Proteasespaltstelle gegen eine Codierungsnucleotidsequenz für eine ubiquitäre Proteasespaltstelle ausgetauscht ist, und wobei durch die zweite genetische Modifizierung die Codierungsnucleotidsequenz für zumindest eines der von dem P-Gen codierten akzessorischen, nicht-strukturellen Proteinen derart modifiziert ist, dass zumindest eines der akzessorischen, nicht-strukturellen Proteine funktionell zerstört ist.

Unter einem "Paramyxovirus" wird ein solches Virus verstanden, das zu der Familie *Paramyxoviridae* gehört und ein Virus, das hieraus durch Propagation hervorgegangen ist. Paramyxoviren umfassen die Subfamilie *Paramyxovirinae* mit den Gattungen Respiroviren, Rubulaviren, Avulaviren und Morbilliviren sowie die Subfamilie *Pneumovirinae* mit den Gattungen Pneumoviren, und Metapneumoviren. Details zur Taxonomie der Paramyxoviren finden sich beispielsweise in Kneipe et al. (2007), Fields Virology, 5. Auflage, Lippincot Williams & Wilkins.

Wie die Erfinder feststellen konnten, eignet sich nach dieser bevorzugten Ausführungsform für die erfindungsgemäße Modifizierung grundsätzlich jeder Stamm des Sendai Virus'. Wichtige besonders geeignete Stämme des Sendai Virus sind "Fushimi", "Harris", "Z-Strain", "Ohita", "Hamamatsu", "Cantell", und "52".

Die Erfinder haben erstmals erkannt, dass eine genetische Modifizierung des F-Gens bzw. des F-Proteins mit Aufhebung der Proteasenrestriktion für eine effiziente Ausbreitung des Virus in Tumorgewebe sorgt und somit ein breites Spektrum unterschiedlicher Tumoren infiziert werden kann.

Die Nukleotidsequenz des F-Gens des Sendai Virus ist in dem beigefügten Sequenzprotokoll unter SEQ ID Nr. 1 wiedergegeben und weist die GeneID 1489775 auf. Die Aminosäuresequenz des F-Proteins des Sendai Virus ist in dem beigefügten Sequenzprotokoll unter SEQ ID Nr. 2 wiedergegeben und weist die Datenbankzugriffsnummern BAA 24390.1 oder NP_056877 auf.

Die Erfinder haben ferner erkannt, dass durch die zweite genetische Modifizierung im P-Gen bzw. P-Protein eine Attenuierung des Virus erreicht wird. Durch die genetische Modifizierung bzw. Mutation im P-Gen, verschieben sich die Leseraster für die Gene bzw. Proteine C', C, Y1, Y2. Das P-Protein bleibt hingegen völlig intakt.

Die Nukleotidsequenz des P-Gens des Sendai Virus ist in dem beigefügten Sequenzprotokoll unter SEQ ID Nr. 3 wiedergegeben. Die Aminosäuresequenz des P-Proteins ist in dem beigefügten Sequenzprotokoll unter SEQ ID Nr. 4 wiedergegeben und weist die Datenbankzugriffsnummern BAA 24386.1 und AAB 06279 auf.

Das P-Gen codiert für verschiedene akzessorische, nicht-strukturelle Proteine mit der Bezeichnung C', C, Y1, Y2, V und W. Diese Proteine entstehen durch überlappende Leserahmen und "RNA-Editing". Die genauen Funktionen der akzessorischen nicht-strukturellen Proteine sind im Detail noch nicht vollständig aufgeklärt, wobei vermutet wird, dass sie in der Interaktion mit dem unspezifischen Abwehrsystem der infizierten Zellen, wie bspw. dem Interferonsystem, interagieren können.

Die erfindungsgemäßen modifizierten Paramyxoviren sind im Gegensatz zu Wildtypviren in der Lage, eigenständig im Tumorgewebe zu replizieren und dieses zu zerstören, wobei keine Beschränkung auf einen bestimmten Tumorzelltyp beobachtet wird. Überraschenderweise ist das erfindungsgemäße Virus jedoch nur noch sehr eingeschränkt in der Lage, in Nicht-Tumorzellen, beispielsweise primären humanen Hepatozyten oder Fibroblasten, zu replizieren. Das erfindungsgemäße Virus infiziert folglich mit sehr hoher Präferenz Tumorzellen gegenüber Nicht-Tumorzellen. Schädigungen des Normalgewebes werden deutlich reduziert, wohingegen die therapeutische Breite des Virus erhöht wird.

Unter "genetischer Modifizierung" wird erfindungsgemäß eine vorzugsweise gezielte Veränderung des Genoms, der genetischen Information bzw. der codierten Proteine des erfindungsgemäßen Paramyxovirus gegenüber dem Wildtyp verstanden, die beispielsweise durch zielgerichtete Mutagenese eingeführt werden kann.

Nach einer bevorzugten Weiterbildung ist die erste genetische Modifizierung derart ausgestaltet, dass diese in einer Tropismuserweiterung resultiert.

Diese Maßnahme hat den Vorteil, dass das erfindungsgemäße Virus von spezifischen Proteasen, die bspw. nur von wenigen Geweben oder Tumorzellen exprimiert werden, unabhängig wird. So ist das Wildtyp-Sendai-Virus bspw. von einer Tryptase abhängig, die nur von Bronchialepithelzellen exprimiert wird und kann deshalb nur diese Zellen infizieren. Das in Kinoh et al. (2009; a.a.O.) beschriebene Virus ist von der Matrix-Metalloproteinase (MMP) abhängig und kann deshalb nur solche Tumorzellen infizieren, die MMP exprimieren. Das weitergebildete erfindungsgemäße Virus hingegen ist durch die erste genetische Modifizierung im F-Gen in der Lage, sämtliche Tumorzellen zu infizieren und zu lysieren.

Nach einer weiteren bevorzugten Ausgestaltung ist die zweite genetische Modifizierung derart ausgestaltet, dass diese in einer Tropismuseinschränkung resultiert.

Diese Maßnahme hat den Vorteil, dass die Virussicherheit erhöht und Nebenwirkungen deutlich reduziert werden. Das erfindungsgemäße Virus lysiert selektiv Tumorzellen, gesunde Zellen hingegen nicht oder nur in sehr begrenztem Maße. Die Erfinder haben erkannt, durch eine gezielte genetische Modifizierung des P-Gens, d.h. eines Nicht-Strukturproteins, die Reaktionsweise der zu lysierenden Zelle so beeinflussen zu können, dass das Virus zielgerichtet in Tumorzellen repliziert. Dieser Ansatz unterscheidet sich von dem von Kinoh et al. (2009; a.a.O.) beschriebenen, bei dem durch eine Deletion des M-Gens, das für ein Strukturprotein codiert, keine vollständigen Viruspartikel mehr synthetisiert werden können. Hierunter leidet jedoch auch die Lyseaktivität. Diese ist bei dem erfindungsgemäßen Virus, beschränkt auf Tumorzellen, überraschenderweise sehr hoch.

Erfindungsgemäß ist durch die erste genetische Modifizierung in dem F-Gen die Codierungsnukleotidsequenz für die SeV-WT-Proteasespaltstelle, die die Aminosäuresequenz VPQSR (SEQ ID Nr. 5) aufweist, gegen eine Codierungsnukleotidsequenz für eine ubiquitäre Proteasespaltstelle, vorzugsweise aus dem F-Gen des Newcastle-Disease-Virus (NDV), die die Aminosäuresequenz RRQKR (SEQ ID Nr. 6) aufweist, ausgetauscht.

Durch die erfindungsgemäße Modifizierung der proteolytischen SeV-WT-Proteasespaltstelle im F-Protein, wird die Proteasenrestriktion des Wildtyps aufgehoben. Das F-Protein kann nunmehr durch ubiquitäre Proteasen gespalten werden, wodurch eine Unabhängigkeit von bestimmten ggf. sich nur im Respirationstrakt von Mäusen befindlichen Proteasen oder aber auch von tumorproduzierenden Proteasen erreicht wird. Das genetisch modifizierte Virus kann dadurch für ein breiteres Spektrum von Tumoren eingesetzt werden, wobei die Gefahr einer Resistenzentstehung deutlich reduziert wird.

Erfindungsgemäß ist durch die zweite genetische Modifizierung im P-Gen die Codierungsnukleotidsequenz für zumindest eines der von dem P-Gen codierten akzessorischen nicht strukturellen Protein derart modifiziert, dass es funktionell zerstört ist.

Diese Maßnahmen haben den Vorteil, dass das erfindungsgemäße genetisch modifizierte Virus derart attenuiert ist, dass die Replikation in nichtmalignen Zellen und damit die Infektion von gesundem Gewebe deutlich eingeschränkt wird. Die erfindungsgemäßen Viren infizieren somit noch zielgerichteter Tumorzellen und können diese lysieren.

Dabei ist es bevorzugt, wenn die akzessorischen nicht-strukturellen Proteine ausgewählt sind aus der Gruppe bestehend aus: C', C, Y1, Y2, V und W, wobei es bevorzugt ist, wenn durch die zweite genetische Modifizierung im P-Gen zumindest die C'/C- und Y1/Y2-Proteine, weiter vorzugsweise zumindest die C'/C-, V- und W-Proteine, weiter vorzugsweise zumindest die C'/C-, V-, W- und Y1/Y2-Proteine modifiziert bzw. funktionell zerstört sind.

Wie die Erfinder in einem Modellsystem feststellen konnten, weisen derart modifizierte Viren eine besonders hohe Selektivität für Tumorzellen und eine besonders schlechte Replikation in Nicht-Tumorzellen auf.

Die Nukleotidsequenz des C'-Gens des Sendai Virus ist in dem beigefügten Sequenzprotokoll unter SEQ ID Nr. 7 wiedergegeben und weist die GeneID AB005796.1 auf. Die Aminosäuresequenz des C'-Proteins des Sendai Virus ist in dem beigefügten Sequenzprotokoll unter SEQ ID Nr. 8 wiedergegeben und weist die Datenbankzugriffsnummer BAA 24394 auf.

Die Nukleotidsequenz des C-Gens des Sendai Virus ist in dem beigefügten Sequenzprotokoll unter SEQ ID Nr. 9 wiedergegeben. Die Aminosäuresequenz des C-Proteins des Sendai Virus ist in dem beigefügten Sequenzprotokoll unter SEQ ID Nr. 10 wiedergegeben und hat die Datenbankzugriffsnummer BAA 24396.

Die Nukleotidsequenz des V-Gens des Sendai Virus ist in dem beigefügten Sequenzprotokoll unter SEQ ID Nr. 11 wiedergegeben. Die Aminosäuresequenz des V-Proteins des Sendai Virus ist in dem beigefügten Sequenzprotokoll unter SEQ ID Nr. 12 wiedergegeben und weist die Datenbankzugriffsnummer BAA 20021 auf.

Die Nukleotidsequenz des W-Gens des Sendai Virus ist in dem beigefügten Sequenzprotokoll unter SEQ ID Nr. 13 wiedergegeben. Die Aminosäuresequenz des W-Proteins des Sendai Virus ist in dem beigefügten Sequenzprotokoll unter SEQ ID Nr. 14 wiedergegeben und weist die Datenbankzugriffsnummer AAX07444 auf.

Die Nukleotidsequenz des Y1-Gens des Sendai Virus ist in dem beigefügten Sequenzprotokoll unter SEQ ID Nr. 15 wiedergegeben. Die Aminosäuresequenz des Y1-Proteins des Sendai Virus ist in dem beigefügten Sequenzprotokoll unter SEQ ID Nr. 16 wiedergegeben und hat die Datenbankzugriffsnummer BAA 24388.

Die Nukleotidsequenz des Y2-Gens des Sendai Virus ist in dem beigefügten Sequenzprotokoll unter SEQ ID Nr. 17 wiedergegeben. Die Aminosäuresequenz des Y1-Proteins des Sendai Virus ist in dem beigefügten Sequenzprotokoll unter SEQ ID Nr. 18 wiedergegeben und hat die Datenbankzugriffsnummer AAX07449.

Nach einer bevorzugten Weiterbildung weist das erfindungsgemäße Virus gegenüber dem Wildtyp (WT) zumindest ein Transgen auf, vorzugsweise ein Suizidgen oder andere Zelltod induzierende oder immunstimulierende Gene.

Diese Maßnahme hat den Vorteil, dass die Zytotoxizität des erfindungsgemäßen Virus für Tumorzellen nochmals erhöht wird, indem das Produkt des Transgens zusätzlich zu einer verstärkten Zerstörung der infizierten Tumorzellen beiträgt. Umfasst sind auch Transgene mit einer Antitumorwirkung.

Vor diesem Hintergrund betrifft ein weiterer Gegenstand der vorliegenden Erfindung eine pharmazeutische Zusammensetzung, die das erfindungsgemäße genetisch modifizierte Paramyxovirus sowie einen pharmazeutisch akzeptablen Träger aufweist.

Pharmazeutisch akzeptable Träger sind im Stand der Technik umfassend beschrieben, beispielsweise in Bauer et al. (1999), Lehrbuch der Pharmazeutischen Technologie, Wissenschaftliche Verlagsgesellschaft mbH Stuttgart, Kibbe et al. (2000) Handbook of Pharmaceutical Excipients, American Pharmaceutical Association and Pharmaceutical Press. Der Inhalt der vorgenannten Publikationen ist Gegenstand der vorliegenden Offenbarung. Es versteht sich, dass die pharmazeutische Zusammensetzung weitere Hilfs- und Wirkstoffe aufweisen kann, wie beispielsweise Zytostatika.

Ein weiterer Gegenstand der vorliegenden Erfindung betrifft die Verwendung des erfindungsgemäßen genetisch modifizierten Paramyxovirus zur therapeutischen und/oder prophylaktischen Behandlung einer Tumorerkrankung, vorzugsweise einer Erkrankung an einem soliden Tumor, weiter vorzugsweise eine Erkrankung an dem hepatozellulären Karzinom.

Mit rund einer Million Neuerkrankungen pro Jahr stellt das hepatozelluläre Karzinom eines der häufigsten Malignome weltweit dar. Nur in wenigen Fällen ist eine kurative Therapie durch Resektion oder Lebertransplantation möglich. Bislang fehlen überzeugende alternative Therapiekonzepte, da eine ausgeprägte Resistenz gegenüber allen bislang getesteten Chemotherapeutika besteht. Die Erfindung schafft hier wirksam Abhilfe.

Ein weiterer Gegenstand der vorliegenden Erfindung betrifft ein Verfahren zur Herstellung einer pharmazeutischen Zusammensetzung zur therapeutischen und/oder prophylaktischen Behandlung einer Tumorerkrankung, vorzugsweise einer Erkrankung an einem soliden Tumor, weiter vorzugsweise eine Erkrankung an dem hepatozellulären Karzinom, das folgende Schritte aufweist: (1) Bereitstellen des erfindungsgemäßen genetisch modifizierten Sendai Virus, und (2) Formulieren des genetisch modifizierten Sendai Virus in einen pharmazeutisch akzeptablen Träger.

Die vorliegende Erfindung wird nun anhand von Ausführungsbeispielen näher erläutert, aus denen sich weitere Merkmale, Vorteile und Eigenschaften der Erfindung ergeben. Dabei wird Bezug genommen auf die beigefügten Abbildungen, in denen Folgendes dargestellt ist:
- Fig. 1: zeigt die genomische Struktur des Sendai Virus;
- Fig. 2: zeigt die Struktur des pSVV10-Plasmides mit der cDNA des Sendai Virus;
- Fig. 3: zeigt die offenen Leseraster (ORFs) der nicht-strukturellen akzessorischen Gene im P-Gen;
- Fig. 4: zeigt die Subklonierung in den Klonierungsvektor pSL1180;
- Fig. 5: zeigt das Prinzip der Mutagenese-PCR;
- Fig. 6: zeigt das Primerschema zur Überprüfung der Mutationen nach der Mutagenese-PCR;
- Fig. 7: zeigt den Schritt 1 der Reklonierung in dem die mutierten *SphI-EcoRI*-Fragmente aus dem pSL1180 Sph1-Eco pSVV10 Klonierungsvektor ausgeschnitten und in einen Sendai Virus Vektor pVV13 einkloniert wurden;
- Fig. 8: zeigt den Schritt 2 der Reklonierung; in den pSVV13 Vektor, dem die ORFs für M, F, HN fehlen; dieser Bereich wurde mit der veränderten F-Spaltstelle aus pRS Id-E Fm über *EcoRI* kloniert; die resultierenden Vektoren beinhalteten den mutierten P/C Bereich und eine ubiquitäre F-Spaltstelle aus NDV;
- Fig. 9: zeigt die Replikation der erfindungsgemäßen Viren auf Vero-Produzentenzellen und Hepatomzellen (Hep3B, HuH7, PLC/PRF/5);
- Fig. 10: zeigt die Replikation der erfindungsgemäßen Viren auf Nicht-Tumorzellen (MRC-5, humane Fibroblasten; PHH, primäre humane Heptozyten);
- Fig. 11: zeigt die Vermehrung der erfindungsgemäßen Viren *in vivo.*

### Ausführungsbeispiele

### 1. Genomischer Aufbau der Sendai Viren

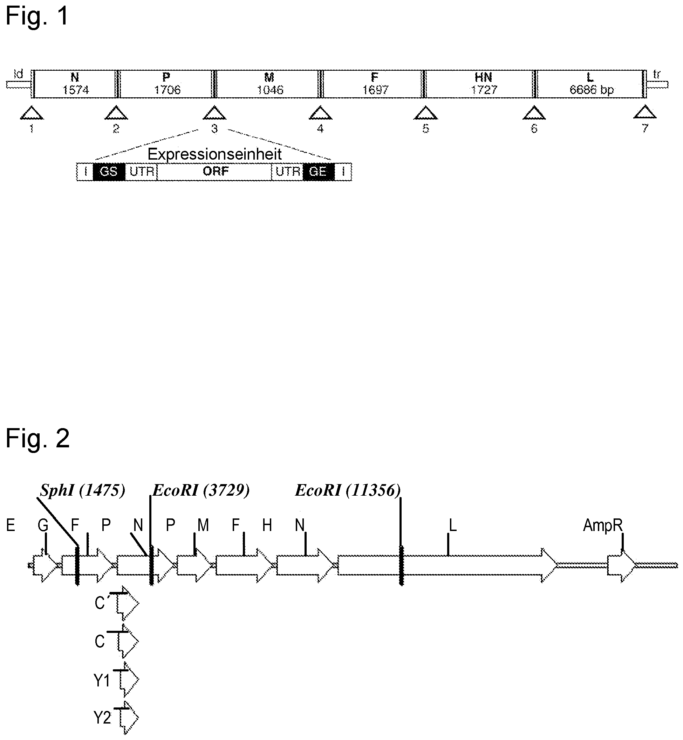

Sendai Viren sind Negativstrang RNA-Viren mit einem Genom von ca. 15 kb. Der genomische Aufbau ist in der Fig. 1 dargestellt. Die Gene der sechs Strukturproteine befinden sich auf der viralen genomischen RNA in der Reihenfolge 3'-N-P-M-F-HN-L-5'. Vor dem ersten Gen befindet sich die 54 Nukleotide umfassende Leader-Region (ld) und hinter dem letzten Gen folgt die 57 Nukleotide lange Trailer-Region (tr). Die Zahlen unter jedem Gen entsprechen der Genlänge in bp. Für die Transkription eines einzelnen Genes startet die virale Polymerase die Synthese der mRNA an einer konservierten Sequenz am 3'-Ende, die als Gene-Start-Motiv (GS) bezeichnet wird, gefolgt von einer nicht-translatierten Region (UTR), dem offenen Leseraster (ORF) des viralen Gens, und letztlich dem Gene-End-Motiv (GE), an dem die mRNA-Synthese anhält. Zwischen zwei Expressionseinheiten findet sich ein konserviertes intergenisches Motiv (I) von 3 bp, das nicht in die mRNA eingebaut wird.

Die Grundlage der Erfindung bildet das sogenannte pSVV10-Plasmid, genauer gesagt das pSVV10IdGFPMFHN-Plasmid mit einer Größe von 19.774 bp, welches für die cDNA des Sendai Virus des Stammes Fushimi, ATCC VR-105, codiert. Das Plasmid ist in der Fig. 2 dargestellt. 2. Detaillierte Darstellung der im P-Gen codierten akzessorischen Proteine.

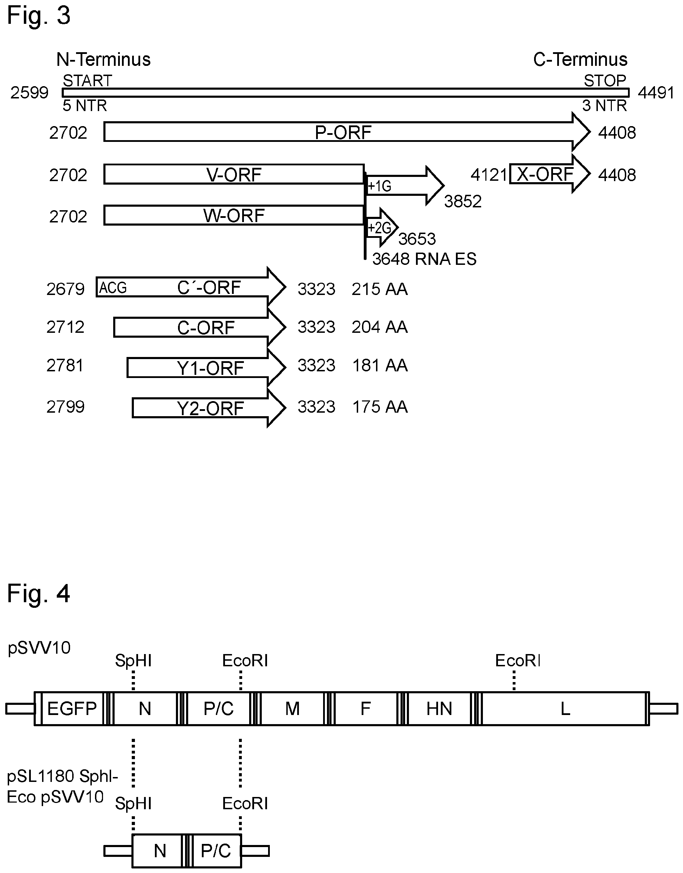

Die akzessorischen Proteine des Sendai Virus C', C, Y1, Y2, V und W werden im P-Gen codiert. Dabei sind die ORFs der C- und Y-Proteine um +1 Base verschoben. Die V- und W-Proteine entstehend je nach Anzahl der inserierten G-Proteine an der sogenannten Editing Site (ES). Der P-ORF-Sequenzbereich mit den akzessorischen Genen ist in der Fig. 3 dargestellt, wobei sich die Zahlen auf die Angaben im PSVV10 Vektor beziehen.

### 3. Subklonierung im Mutagenese-Plasmid

Zum Einfügen von Punktmutationen in den Genen der akzessorischen Proteine wurde eine Mutagenese-PCR durchgeführt. Für diese Methode können allerdings nur Plasmide mit einer maximalen Größe von 8 kb verwendet werden. Der P/V/C-Bereich des Genoms des Sendai Virus wurde aus dem Sendai-Virus-Vektor pSVV10 (19774 bp) über die Restriktionsenzyme *EcoRI* und *SphI* in den Klonierungsvektor pSL1180 (3422 bp) subkloniert. Hierzu wurden die beiden Vektoren pSVV10 (Fig. 2) und pSL1180 mit den Enzymen *EcoRI* und *SphI* verdaut und der zu mutierende Bereich aus pSVV10 (2254 bp) mit dem Vektorrückgrat aus sPL1180 (3303 bp) ligiert. Das resultierende Klonierungsplasmid pSL1180 SphI-Eco pSVV10 hat eine Größe von 5557 bp; vgl. Fig. 4.

### 4. Mutagenese

Durch gezielte Mutation soll die Transkription der akzessorischen Gene C', C, Y1 und Y2 verhindert werden. Auch eine Mutation in der Editing-Site führt dazu, dass das Editing nicht mehr durchgeführt werden kann. Im folgenden Überblick ist der relevante Klonierungsbereich von pSL1180 mit allen Gen-Starts und der Editing-Site dargestellt (SEQ ID NR. 19):
ACG = Start C' Leserahmen von P/W/V +1
ATG = Start P/W/V Leserahmen 1
ATG = Start C Leserahmen von P/W/V +1
ATG = START Y1 (STOP BEI 647) Leserahmen von P/W/V +1

Es wurden drei spezielle Primer entwickelt, die einerseits die Startsequenzen der Gene für die akzessorischen Proteine funktionell zerstören, die aber andererseits im P-Leserahmen keine Aminosäure-Änderung hervorrufen.

### Primer 1 (SeV Cₖₒ):

5' CGCATGGATCAAGACGCCTTCATTCTAAAAGAAGATTCTGAAGTAGAGAGG 3' (SEQ ID NR. 20)
**Unveränderte P-Aminosäuren:**
**Mutierte C-Aminosäuren:**

### Primer 2 (SeV Yₖₒ):

5' CTCTCGGACGTTATCGGATTCCTCGACGCTGTCCTG 3' (SEQ ID NR. 24)
**Unveränderte P-Aminosäuren:**
**Mutierte Y-Aminosäuren:**

### Primer 3 (SeV Vₖₒ):

5' GACTCAACAAAGAAAGGCATAGGTGAGAACACATCATCTATG 3' (SEQ ID NR. 28)
Unveränderte P-Aminosäuren:
**Mutierte Aminosäuren von V und W:**

Mit den Primern wurden in der Mutagenese-PCR folgende Mutationen hergestellt: Gewünschte Mutationen:

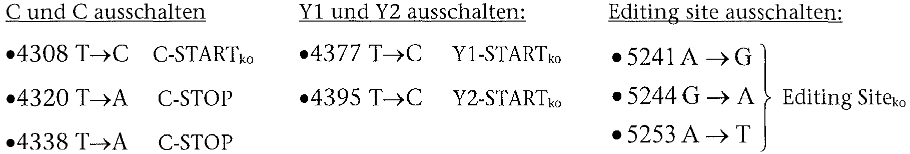

Das "QuickChange Multi Site directed Kit" der Firma Stratagene® ermöglicht ein gezieltes Einfügen von Punktmutationen bei Plasmiden mit bis zu 8 kb Größe in drei aufeinanderfolgenden Schritten. Im ersten Schritt lagern sich die zur Reaktion gegebenen Fehlpaarungsprimer, die einzelne Punktmutationen enthalten, an den denaturierten Template-Einzelstrang an. Dabei ist darauf zu achten, dass alle Primer an denselben Template-Strang binden. Die *PfuTurbo*-Polymerase verlängert von den Primern aus die komplementäre Sequenz, ohne die Primer zu verdrängen. Der neu generierte DNA-Strang beinhaltet nun die Mutationen und einzelne überhängende Enden, sogenannte "nicks", die von Komponenten des Enzym Blends passend verschoben werden.

Im zweiten Schritt erfolgt ein Verdau mit *DpnI,* wodurch spezifisch methylierte und hemimethylierte DNA verdaut wird. Da Plasmide, die in *Escherichia coli* vermehrt wurden, *dam* methyliert sind, wird nur das parentale Template verdaut, nicht aber die in der PCR entstandenen Kopien, welche die Mutationen enthalten.

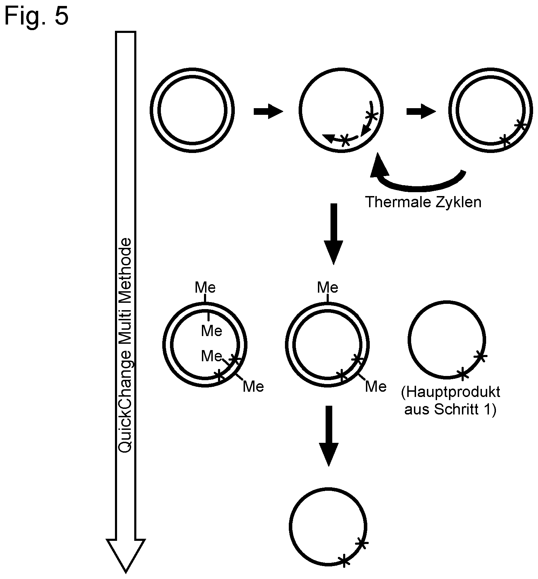

Im letzten Schritt wird die ssDNA in XL10-Gold-Ultrakompetente Zellen transformiert und dort *in vivo* in dsDNA konvertiert. Die Plasmide können nun aus den Bakterien isoliert und auf eingefügte Mutation mittels Sequenzierung analysiert werden. Das Prinzip der Mutagenese PCR ist in der Fig. 5 dargestellt.

Der Mutageneseansatz setzt sich wie folgt zusammen (Tab. 1):

**Tabelle 1: Ansatz der SeV Mutagenese PCR zur Generierung rekombinanter Sendai Viren mit partiellen Deletionen in den akzessorischen Proteinen**

| Komponenten | Vko | Cko/Yko | Cko/Yko/Vko |
|---|---|---|---|
| 10 x Quick Change Puffer | 2,5 µl | 2,5 µl | 2,5 µl |
| Quick Solution | 0,75 µl | 0,75 µl | 0,75 µl |
| dNTP Mix | 1 µl | 1 µl | 1 µl |
| QuickChange Mutli Enzyme Blend | 1 µl | 1 µl | 1 µl |
| Plasmid | 100 ng | 100 ng | 100 ng |
| Primer SeV Vₖₒ | 0,9 µl | - | 0,9 µl |
| Primer SeV Cₖₒ | - | 0,9 µl | 0,9 µl |
| Primer SeV Yₖₒ | - | 0,9 µl | 0,9 µl |
| H₂O | ad 25 µl | ad 25 µl | ad 25 µl |

Die Mutagenese-PCR ähnelt einer herkömmlichen PCT, nur die Extensionszeit ist sehr lang, da der komplette Vektor komplementiert werden muss und variiert somit für jeden Vektor: Zwei Minuten pro kb; hier: pSL1180 + Eco-SphI Fragment aus pSVV10 ∼5,5 kb entspricht 11 Minuten Extension; vgl. Tab. 2:

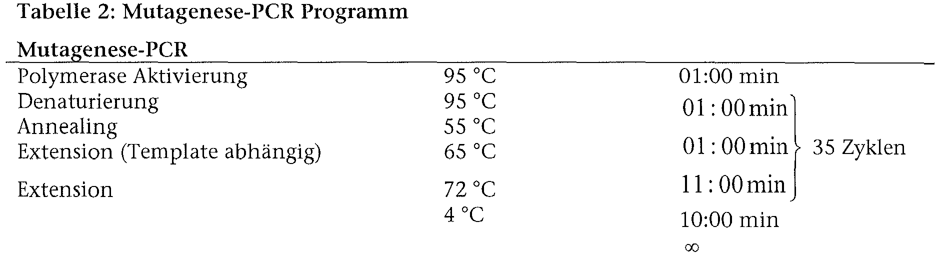

Direkt nach Amplifizierung wurde 1 µl *DpnI* Restriktionsenzym (10 U/µl) zu dem PCR-Ansatz gegeben, mit der Pipette resuspendiert, kurz zentrifugiert und eine Stunde bei 37 °C verdaut. Die entstandene ssDNA wurde in XL10-Gold ultrakompetente Zellen transformiert und mittels Miniprep als dsDNA-mutiertes Plasmid aus den Bakterien isoliert; vgl. Tab. 3:

**Tabelle 3: Veränderte Sequenz durch Mutagenese; * Kurutani et al. Genes to Cells, 1998 $ Gotoh et al. FEBS letters, 1999**

| | C START | C START | C-STOP | | C-STOP |
|---|---|---|---|---|---|
| WT | //ACG-// | -/-ATG-/- | -/-TTA-/- | | -/-TTG-// |
| C' und C (-) | | -C- | -A- | | -A- |
| Kurotani* | | | -A- | | -A- |
| Gotoh^{$} | G-- | | -A- | | -A- |

| | Y1 START | | Y2 START | | |
|---|---|---|---|---|---|
| WT | //ATGTTA-//-------------- | | /-ATG-// | | |
| Y1 und Y2 (-) | -C- | | -C- | | |
| Kurotani* | -C--A- | | -C- | | |
| Gotoh^{$} | -C--A- | | -C- | | |

| | Editing site ko | | STOP | STOP | |
|---|---|---|---|---|---|
| WT | //ACAAAAAAG GGC ATA GGA GAG | | | | |
| Editing site | --G - - A | | -- T | | |
| +1G | | | | -TGA- | |
| +2G | | | -TGA- | | |
| Kurotani* | | | | | |
| Gotoh^{$} | --G- - A | | | | |

### 5. Sequenzierung

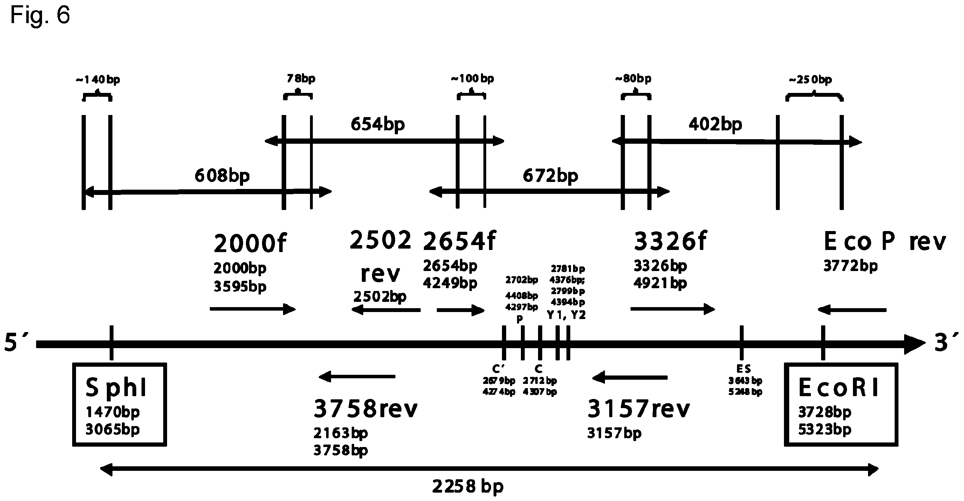

Die entstandenen mutierten Plasmide wurden mittels Sequenzierung auf das korrekte Einfügen der Mutationen überprüft. Ein Primerschema mit der Abdeckung des kompletten Mutationsbereichs in der Fig. 6 dargestellt. Die oberen Zahlen betreffen Angaben für Positionen im Originalvektor pSVV10 (∼19kb), die unteren Zahlen betreffen Angaben für Positionen im pSL1180+pSVV10 Klonierungsvektor (∼5,5kb).

### 6. Reklonierung der mutierten Sequenzen in einem für das komplette Sendai Virus codierenden Vektor

Der mutierte Sequenzbereich musste wieder zurück in einen Vektor mit kompletter cDNA-Sequenz des Sendai Virus kloniert werden. Da Viren hergestellt werden sollten, die statt der nur durch die Tryptase "Clara" im respiratorischen Trakt von Nagetieren aktivierbaren Spaltstelle eine ubiquitäre F-Spaltstelle haben sollten, wurde der Eco-Eco-Bereich aus dem Vektor pRS Id-EGFP Fmut (19958 bp, Sascha Bossow, MPI München) verwendet. Dieser beinhaltet statt der Spaltstelle im F-Protein des Sendai Virus mit der Nukleotidsequenz (GTTCCACAGTCGAGA; SEQ ID Nr. 33) eine ubiquitäre Spaltstelle für das F-Protein aus dem Newcastle-Disease-Virus mit der Sequenz (CGTCGTCAGAAGAGA; SEQ ID Nr. 34).

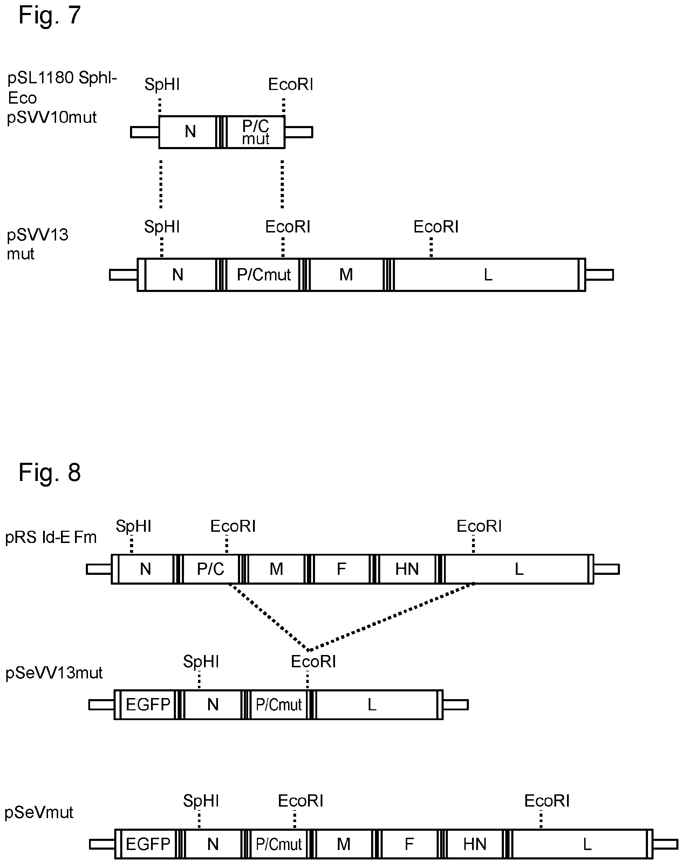

Die verschiedenen mutierten *SphI-EcoRI*-Fragmente wurde zunächst aus dem pSL1180 Sph1-Eco pSVV10 Klonierungsvektor ausgeschnitten und in einen Sendai Virus Vektor pSVV13 kloniert, der dem Vektor pSVV10 gleicht, dem aber die Bereiche für das F-Gen und HN-Gen fehlen. In diesen Vektor wurden dann die fehlenden Bereiche für das F-Gen und HN-Gen mit der veränderten Spaltstelle im F-Gen aus pRS Id-EGFP Fut über *EcoRI* kloniert. Resultierenden Vektoren wurden als pSeVmut bezeichnet. Funktionelle Sendai Viren werden anhand der "rescue"-Methode über die Transfektion von BSR-T7-Zellen hergestellt. Schritt 1 der Reklonierung ist in der Fig. 7 und Schritt 2 der Reklonierung in der Fig. 8 dargestellt.

### 7. Herstellung rekombinanter Sendai Viren

Durch die Methode des "rescue" für Paramyxoviridae ist es möglich, genetisch veränderte Viren herzustellen. Dazu wurden BSR-T7 Zellen, welche konstitutiv die T7 RNA-Polymerase exprimieren, durch Lipofektion mit Helferplasmiden und für die cDNA des Sendai Virus codierenden Plasmide cotransfiziert. Die unter einem T7 Promotor stehenden Plasmide werden in der Zelle transkribiert, so dass durch mehrere Schritte virale Proteine und virale Negativ-Strang-RNA-Genome gebildet werden und funktionelle Viren entstehen.

BSR-T7 Zellen (3x10⁵ pro Kavität einer Platte mit sechs Kavitäten) wurden ausgesät und über Nacht bei 37 °C kultiviert. Zur Transfektion wurden in einem Röhrchen 200 µl DMEM mit FuGENE 6 vorgelegt (Menge an Fugene 6 entsprach einem Verhältnis von 2 µl pro µg DNA) und für fünf Minuten inkubiert. Nach Hinzufügen der DNA Komponenten, die in der Tab. 4 gelistet sind, erfolgte ein weiterer Inkubationsschritt für 25 Minuten bei Raumtemperatur.

**Tabelle 4: DNA Komponenten eines "rescue" Ansatzes**

| Komponente | DNA Menge |
|---|---|
| SeV cDNA | 7,5 µg |
| pTM-N | 250 ng |
| pTM-P/C⁻ | 150 ng |
| pTM-L | 50 ng |

Die BSR-T7 Zellen wurden zweimal mit DMEM gewaschen; danach wurde 1,8 ml Medium DMEM + 2% FCS vorgelegt. Das inkubierte Transfektionsgemisch wurde tropfenweise unter Schwenken hinzugegeben und die Zellen für drei Tage bei 33 °C inkubiert. Danach wurden die transfizierten BSR-T7 Zellen dreimal mit je 1 ml DMEM gewaschen, um Plasmidreste zu entfernen. Zu jedem Ansatz wurde 1 ml frisches Medium DMEM + 2% FCS gegeben und die neu entstandenen Viren nach einem Tag geerntet.

Vor der Übertragung zur Vermehrung auf Vero-Zellen wurde der virushaltige Überstand bei 300 rpm für vier Minuten bei Raumtemperatur zentrifugiert. Vier Tage vorher vorbereitete Vero-Zellen (ausgelegt: 2x10⁵ Zellen pro 3,5 cm Schale; Soll: ca. 10⁶ Zellen pro 3,5 cm Schale am Tag der Infektion) wurden zweimal mit DMEM gewaschen und mit 100 bis 500 µl BSR-T7-Überstand (ad 500 µl Adsorptionsvolumen mit Kulturmedium) für eine Stunde bei 33 °C unter Schwenken (alle 15 Minuten) infiziert. Das Inokulum wurde abgenommen, die Zellen zweimal mit DMEM gewaschen und in 1 ml Kulturmedium für zwei bis fünf Tage unter täglichem Mediumswechsel bei 33 °C inkubiert. Sobald im Fluoreszenzmikroskop eGPF als viral codiertes Markerprotein sichtbar war, wurde der Kulturüberstand abgenommen. Mit dieser initialen Viruspassage wurden weitere Passagen (Passage zwei und drei) in größerem Maßstab hergestellt. Die Titer der Virusnachkommen wurden mittels der TCID₅₀-Methode quantifiziert.

Folgende erfindungsgemäße genetisch modifizierte bzw. rekombinante Sendai Viren wurden hergestellt:
a) SeV Fmut: Sendai Virus Stamm Fushimi mit NDV-Spaltstelle
b) SeV Fmut dV: wie a, zusätzlich mit Mutationen in dem V- und W- Gen
c) SeV Fmut dC: wie a, zusätzlich mit Mutationen in den C- und C'-Genen
d) SeV Fmut dCdY: wie a, zusätzlich mit Mutationen in den C- und C'-Genen und Y1- und Y2-Genen
e) SeV Fmut dCdV: wie a, zusätzlich mit Mutationen in den C- und C'-Genen, V- und W-Genen
f) SeV Fmut dCdYdV: wie a, zusätzlich mit Mutationen in den C- und C'-Genen, V-, W- und Y1- und Y2-Genen

### 8. Charakterisierung der rekombinanten Sendai Viren

Die hergestellten rekombinanten Sendai Viren wurden ausführlich auf mehreren Ebenen charakterisiert. So wurde die Virusreplikation auf nichttransformierten Vero-Produzentenzellen sowie auf Tumorzellen, im Speziellen auf Hepatomzellen (Hep3B, HuH7, PLC/PRF/5), sowie auf weitere Nicht-Tumorzellen, wie der humanen Fibroblastenzelllinie MRC-5 und primären humanen Hepatozyten (PHH) unterschiedlicher Spender untersucht.

### Wachstumskurven

1. 1x10⁵ Zellen pro 12 Well in 500 µl auslegen (Vero, Hepatomzelle, MRC-5, PHH werden geliefert, ebenfalls von 1x10⁵ Zellen ausgehen)
2. ÜN anwachsen lassen
3. Medium abnehmen, 1x Waschen mit PBS
4. +250 µl Optimem
5. Virus in Optimem verdünnen (MOI 0,05)
6. Verdünntes Virus zu den Zellen geben
7. mit allen 6 SeV Varianten infizieren (Rest der Verdünnungen einfrieren und als Startwert titrieren)
8. 1 h bei 37 °C infizieren
9. Zellen 2x Waschen (PBS)
10. +1 ml frisches Medium (DMEM 5% FCS)
11. nach 24, 48, 72 h, 96 h (jeweils nach Infektionsstart) Viren im ÜS abnehmen und bei -80°C lagern (je 200 µl)
   a. 2x vorsichtig Waschen mit Medium
   b. +1000 ml Medium (DMEM 5% FCS), Zellen darin abschaben
12. Lysate bei -80 °C einfrieren
13. Für Titration auftauen
   a. 2 Min Wasserbad 37 °C
   b. 10-15 sec vortexten
   c. 2 Minuten 3000 x g kleine Zentrifuge
14. runterzentrifugieren
15. Titration auf Veros (TCID₅₀)
   d. Virusverdünnungen herstellen (erste Reihe immer unverdünnt)
   e. Zu 96 Well Platten geben (immer frische Patte auf 4 °C stellen)
   f. Zellen trypsinieren
   g. Zählen und 2x10⁴ Zellen /96 Well dazugeben
   h. Nach 3 Tagen Titer auswerten

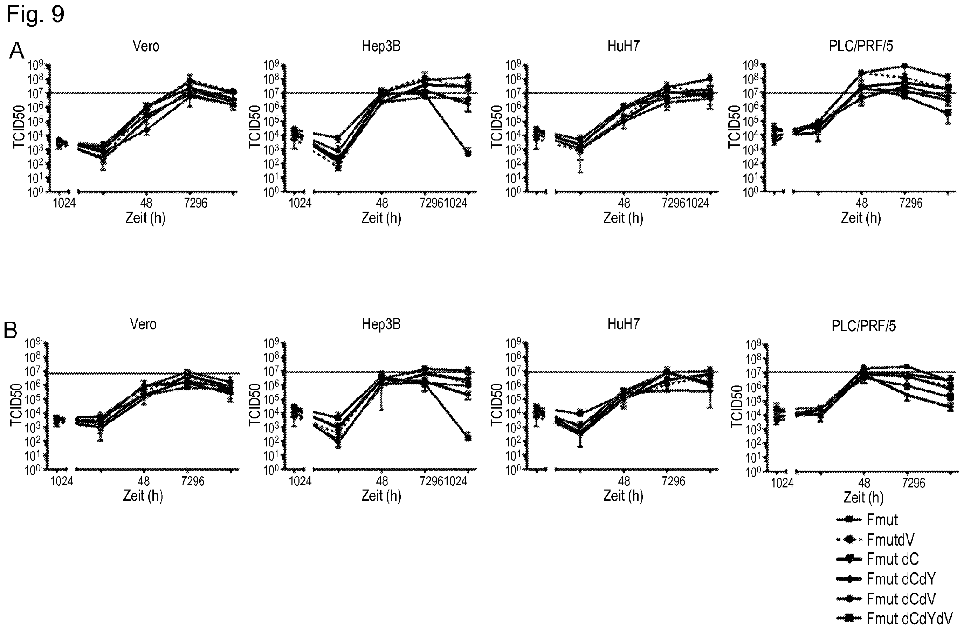

In der Fig. 9 ist die Virusreplikation auf den Vero-Produzentenzellen und den Hepatomzellen dargestellt. Dabei zeigt sich eine ausgesprochen gute Replikation in den Vero-Produzentenzellen mit vergleichbaren Titern für alle generierten rekombinanten Viren mit einer Virusausbeute zwischen 10⁷ und 10⁸ TCID₅₀/ml.

Die Titration der Viruspartikel wurde in drei unabhängigen Versuchen wiederholt, angegeben sind der Mittelwert und die Standardabweichung (IO = Inokulum).

In den drei humanen Standardzelllinien für das hepatozelluläre Karzinom HuH7, PLC/PRF/5 und Hep3B konnte gezeigt werden, dass alle rekombinanten Viren eine ausgesprochen gute Replikation in Tumorzellen aufweisen. Somit ist eine entscheidende Voraussetzung für eine relevante onkolytische Aktivität der erfindungsgemäßen rekombinanten Viren erfüllt.

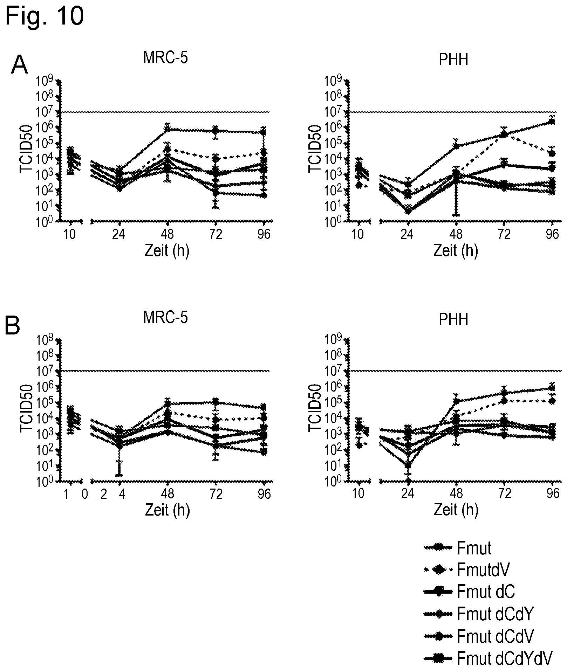

Als Nicht-Tumorzellen wurden exemplarisch die humane Fibroblastenzelllinie MRC-5 und primäre humane Hepatozyten (PHH) von meherern unterschiedlichen Spendern untersucht. Das Ergebnis ist in Fig. 10 dargestellt. Die Intensität der Onkolyse erfolgt bei den untersuchten Varianten in Abhängigkeit der Deletionen in den akzessorischen Proteinen. Viren mit einer einfachen Deletion (SeV V Fmut dC, SeV V Fmut dV) haben ihre Fähigkeit, in Normalzellen zu replizieren, nur moderat verloren, wohingegen Viren mit zwei oder mehr Deletionen (SeV Fmut dCdY, SeV Fmut dCdV, SeV Fmut dCdYdV) effektiv zwar in Hepatomzellen, also Tumorzellen, replizieren und diese zerstören, Infektionen in Normalzellen aber kaum zu beobachten sind.

Die Titration der Viruspartikel wurde in drei unabhängigen Versuchen (bei PHH drei unterschiedliche Spender) wiederholt. Angegeben sind der Mittelwert und die Standardabweichung (IO = Inokulum).

In einem weiteren Experiment wurde die Virusausbreitung *in vivo* untersucht.
1. Balb c nu/nu Mäusen wurden 5x10⁶ HuH7 Tumorzellen subcutan unter die rechte Flanke implantiert
2. Bei Erreichen eines Tumorvolmens von mindestens 100 mm³ wurde Sendai Virus in 100 µl PBS intratumoral appliziert.
3. 2 Tage nach Virusinjektion wurden die Tiere getötet und die Tumore entnommen.
4. Ein Teil der Tumore wurde in Tissue Tack eingebettet, eingefroren und mit einem Kryotom geschnitten. Im Fluoreszenzmikroskop wurde das von Virus exprimierte GFP direkt nachgeweisen.
5. Ein anderer Teil der Tumore wurde in Paraffin eingebettet und GFP mit spezifischen anti GFP Antikörpern nachgewiesen

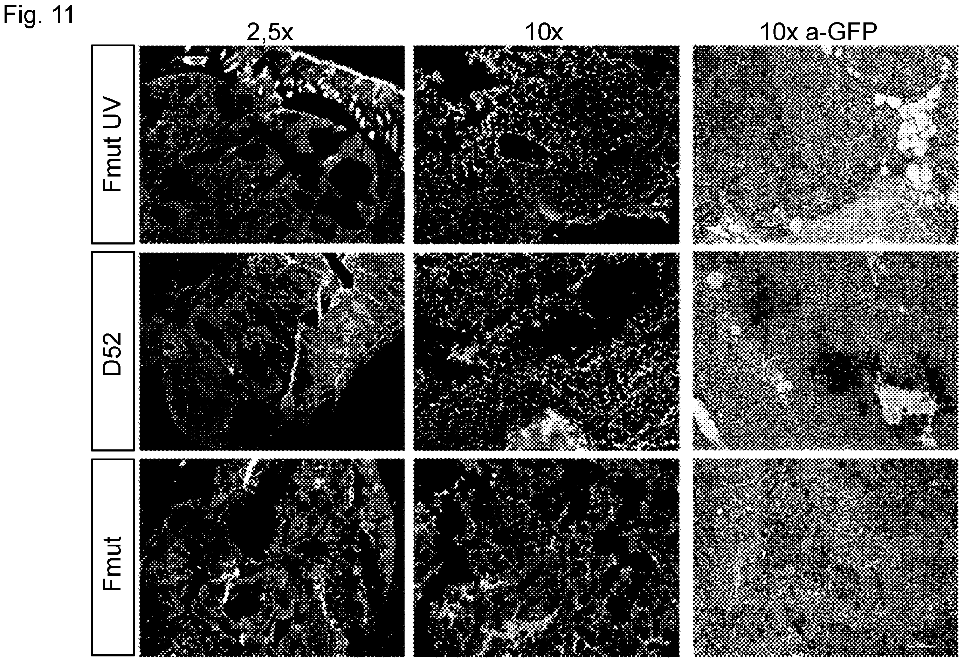

Das Ergebnis ist in der Fig. 11 dargestellt. Es konnte *in vivo* nachgewiesen werden, dass die Tumorzellen durch das rekombinante Virus SeV Fmut infiziert werden und das Virus in der Lage ist, sich dort auszubreiten. Dieser Befund bestätigt, dass eine Replikation *in vivo* außerhalb der Lunge, wie gewünscht, in Tumorgewebe möglich ist. SeV Fmut Viren sind in der Lage, sich in humanem Hepatom-Xenograft-Gewebe (HuH7) zu vermehren, D52-Viren ("Fushimi"-Wildtypvarianten) bleiben nach der intratumoralen Virusapplikation lokal stark begrenzt.

### 9. Fazit

Die Erfinder konnten anhand von verschiedenen rekombinanten Sendai Viren, die in ihrem F-Gen und ihrem P-Gen gegenüber dem Wildtyp genetisch modifiziert wurden, zeigen, dass diese als onkolytische Viren in der Antitumortherapie eingesetzt werden können.

Folgende Sequenzen sind gelistet:
SEQ ID Nr. 1: Nukleotidsequenz des F-Gens von SeV (Stamm "Ohita")
SEQ ID Nr. 2: Aminosäuresequenz des F-Proteins von SeV (Stamm "Ohita")
SEQ ID Nr. 3: Nukleotidsequenz des P-Gens von SeV (Stamm "Ohita")
SEQ ID Nr. 4: Aminosäuresequenz des P-Proteins von SeV (Stamm "Ohita")
SEQ ID Nr. 5 Aminosäuresequenz der SeV-WT-Proteasespaltstelle
SEQ ID Nr. 6: Aminosäuresequenz der Proteasespaltstelle aus dem F-Protein von NDV
SEQ ID Nr. 7: Nukleotidsequenz des C'-Gens von SeV (Stamm "Ohita")
SEQ ID Nr. 8: Aminosäuresequenz des C'-Proteins von SeV (Stamm "Ohita")
SEQ ID Nr. 9: Nukleotidsequenz des C-Gens von SeV (Stamm "Ohita")
SEQ ID Nr. 10: Aminosäuresequenz des C-Proteins von SeV (Stamm "Ohita")
SEQ ID Nr. 11: Nukleotidsequenz des V-Gens von SeV (Stamm "Hamamatsu")
SEQ ID Nr. 12: Aminosäuresequenz des V-Proteins von SeV (Stamm "Hamamatsu")
SEQ ID Nr. 13: Nukleotidsequenz des W-Gens von SeV (Stamm "Cantell")
SEQ ID Nr. 14: Aminosäuresequenz des W-Proteins von SeV (Stamm "Cantell")
SEQ ID Nr. 15: Nukleotidsequenz des Y1-Gens von SeV (Stamm "Ohita")
SEQ ID Nr. 16: Aminosäuresequenz des Y1-Proteins von SeV (Stamm "Ohita")
SEQ ID Nr. 17: Nukleotidsequenz des Y2-Gens von SeV (Stamm "52")
SEQ ID Nr. 18: Aminosäuresequenz des Y2-Proteins von SeV (Stamm "52")
SEQ ID Nr. 19: Nukleotidsequenz des Klonierungsbereich von pSL1180
SEQ ID Nr. 20: Nukleotidsequenz PCR-Primer 1
SEQ ID Nr. 21: Nukleotidsequenz P-Gen
SEQ ID Nr. 22: Nukleotidsequenz P-Gen mut.
SEQ ID Nr. 23: Nukleotidsequenz C-Gen mut.
SEQ ID Nr. 24: Nukleotidsequenz PCR-Primer 2
SEQ ID Nr. 25: Nukleotidsequenz P-Gen
SEQ ID Nr. 26: Nukleotidsequenz Y1-Gen
SEQ ID Nr. 27: Nukleotidsequenz Y1-Gen mut.
SEQ ID Nr. 28: Nukleotidsequenz PCR-Primer 3
SEQ ID Nr. 29: Nukleotidsequenz P-Gen
SEQ ID Nr. 30: Nukleotidsequenz P-Gen mut.
SEQ ID Nr. 31: Nukleotidsequenz V-Gen mut.
SEQ ID Nr. 32: Nukleotidsequenz W-Gen mut.
SEQ ID Nr. 33: Nukleotidsequenz der Spaltstelle im F-Gen von SeV
SEQ ID Nr. 34: Nukleotidsequenz der Spaltstelle im F-Gen von NDV

## Patentansprüche

1. Genetisch modifiziertes Sendai Virus (SeV), das gegenüber dem Wildtyp (WT) in seinem F-Gen zumindest eine erste genetische Modifizierung und in seinem P-Gen zumindest eine zweite genetische Modifizierung aufweist, wobei durch die erste genetische Modifizierung die Codierungsnucleotidsequenz für die SeV-WT-Proteasespaltstelle gegen eine Codierungsnucleotidsequenz für eine ubiquitäre Proteasespaltstelle ausgetauscht ist, und wobei durch die zweite genetische Modifizierung die Codierungsnucleotidsequenz für zumindest eines der von dem P-Gen codierten akzessorischen, nicht-strukturellen Proteinen derart modifiziert ist, dass zumindest eines der akzessorischen, nicht-strukturellen Proteine funktionell zerstört ist.

2. Genetisch modifiziertes Sendai Virus nach Anspruch 1, **dadurch gekennzeichnet, dass** die ausgetauschte ubiquitäre Proteasespaltstelle aus dem F-Gen des Newcastle-Disease-Virus (NDV) stammt.

3. Genetisch modifiziertes Sendai Virus nach Anspruch 1, **dadurch gekennzeichnet, dass** die SeV-WT-Proteasespaltstelle die Aminosäuresequenz VPQSR (SEQ ID Nr. 5) und/oder die ubiquitäre Proteasespaltstelle die Aminosäuresequenz RRQKR (SEQ ID Nr. 6) aufweisen.

4. Genetisch modifiziertes Sendai Virus nach einem der vorherigen Ansprüche, **dadurch gekennzeichnet, dass** zumindest eines der akzessorischen, nicht-strukturellen Proteine nicht transkribierbar ist.

5. Genetisch modifiziertes Sendai Virus nach einem der vorherigen Ansprüche, **dadurch gekennzeichnet, dass** in zumindest einem der akzessorischen, nicht-strukturellen Proteine das Start-Codon funktionell zerstört ist.

6. Genetisch modifiziertes Sendai Virus nach Anspruch 1, **dadurch gekennzeichnet, dass** die akzessorischen, nicht-strukturellen Proteine ausgewählt sind aus der Gruppe bestehend aus: C', C, Y1, Y2, V und W.

7. Genetisch modifiziertes Sendai Virus nach Anspruch 6, **dadurch gekennzeichnet, dass** durch die zweite genetische Modifizierung zumindest die C- (C und/oder C') und Y- (Y1 und/oder Y2) Proteine modifiziert, vorzugsweise funktionell zerstört sind.

8. Genetisch modifiziertes Sendai Virus nach Anspruch 6, **dadurch gekennzeichnet, dass** durch die zweite genetische Modifizierung zumindest die C- (C und/oder C'), V- und W-Proteine modifiziert, vorzugsweise funktionell zerstört sind.

9. Genetisch modifiziertes Sendai Virus nach Anspruch 6, **dadurch gekennzeichnet, dass** durch die zweite genetische Modifizierung zumindest die C- (C und/oder C'), V-, W- und Y- (Y1 und/oder Y2) Proteine modifiziert, vorzugsweise funktionell zerstört sind.

10. Genetisch modifiziertes Sendai Virus nach einem der vorherigen Ansprüche, das gegenüber dem Wildtyp (WT) zumindest ein Transgen aufweist, welches vorzugsweise Suizidgen oder ein anderes Zelltod induzierendes oder immunstimulierendes Gen ist.

11. Pharmazeutische Zusammensetzung, die das genetisch modifizierte Sendai Virus nach einem der Ansprüche 1 bis 10 und einen pharmazeutisch akzeptablen Träger aufweist.

12. Verwendung eines genetisch modifizierten Sendai Virus zur Herstellung eines Arzneimittels zur therapeutischen und/oder prophylaktischen Behandlung einer Tumorerkrankung, vorzugsweise einer Erkrankung an einem soliden Tumor, weiter vorzugsweise einer Erkrankung an dem hepatozellulären Karzinom, **dadurch gekennzeichnet, dass** das genetisch modifizierte Sendai Virus das genetisch modifizierte Sendai Virus nach einem der Ansprüche 1 bis 10 ist.

13. Verfahren zur Herstellung einer pharmazeutischen Zusammensetzung zur therapeutischen und/oder prophylaktischen Behandlung einer Tumorerkrankung, vorzugsweise einer Erkrankung an einem soliden Tumor, weiter vorzugsweise einer Erkrankung an dem hepatozellulären Karzinom, das folgende Schritte aufweist:
(1) Bereitstellen des genetisch modifizierten Sendai Virus nach einem der Ansprüche 1 bis 10 und
(2) Formulieren des genetisch modifizierten Sendai Virus in einen akzeptablen Träger.

## Claims

1. A genetically modified Sendai Virus (SeV) which, in comparison with the wild type (wt), comprises in its F gene at least a first genetic modification and in its P gene at least a second genetic modification, wherein by the first genetic modification the nucleotide sequence coding for the SeV wt protease cleavage site is replaced by a nucleotide sequence coding for a ubiquitous protease cleavage site, and wherein by the second genetic modification the coding nucleotide sequence of at least one of the accessory non-structural proteins encoded by the P gene is modified in such a manner that at least one of the accessory non-structural proteins is functionally destroyed.

2. The genetically modified Sendai Virus of claim 1, **characterized in that** the ubiquitous protease cleavage site originates from the F gene of the Newcastle disease virus (NDV).

3. The genetically modified Sendai Virus of claim 1, **characterized in that** the SeV wt protease cleavage site comprises the amino acid sequence VPQSR (SEQ ID No. 5) and/or the ubiquitous protease cleavage site comprises the amino acid sequence RRQKR (SEQ ID No. 6).

4. The genetically modified Sendai Virus of any of the preceding claims, **characterized in that** by the second genetic modification at least one of the accessory non-structural proteins is non-transcribable.

5. The genetically modified Sendai Virus of any of the preceding claims, **characterized in that** in at least one of the accessory, non-structural proteins the start codon is functionally destroyed.

6. The genetically modified Sendai Virus of claim 1, **characterized in that** the accessory non-structural proteins are selected from the group consisting of: C', C, Y1, Y2, V and W.

7. The genetically modified Sendai Virus of claim 6, **characterized in that** by the second genetic modification at least the C (C and/or C') and Y (Y1 and/or Y2) proteins are modified, preferably functionally destroyed.

8. The genetically modified Sendai Virus of claim 6, **characterized in that** by the second genetic modification at least the C (C and/or C'), V and W proteins are modified, preferably functionally destroyed.

9. The genetically modified Sendai Virus of claim 6, **characterized in that** by the second genetic modification at least the C (C and/or C'), V, W and Y (Y1 and/or Y2) proteins are modified, preferably functionally destroyed.

10. The genetically modified Sendai Virus of any of the preceding claims, which, in comparison with the wild type (wt), comprises at least one transgene, which preferably is a suicide gene or another gene that induces cell death or an immunostimulating gene.

11. A pharmaceutical composition comprising the genetically modified Sendai Virus of any of claims 1 to 10 and a pharmaceutically acceptable carrier.

12. A use of a genetically modified Sendai Virus for manufacturing a medicament for the therapeutical and/or prophylactical treatment of a tumor disease, preferably an affliction with a solid tumor, further preferably an affliction with a hepatocellular carcinoma, **characterized in that** the genetically modified Sendai Virus is the genetically modified Sendai Virus of any of claims 1 to 10.

13. A method for the production of a pharmaceutical composition for the therapeutical and/or prophylactical treatment of a tumor disease, preferably of an affliction with a solid tumor, further preferably an affliction with the hepatocellular carcinoma, comprising the following steps:
(1) Providing the genetically modified Sendai Virus of any of claims 1 to 10, and
(2) Formulating the genetically modified Sendai Virus into an acceptable carrier.

## Revendications

1. Virus Sendai (SeV) génétiquement modifié, qui présente, par rapport au type sauvage (WT), dans son gène F, au moins une première modification génétique et, dans son gène P, au moins une deuxième modification génétique, dans lequel la séquence nucléotidique de codage pour le site de clivage à la protéase de SeV-WT est remplacée, par la première modification génétique, par une séquence nucléotidique de codage pour un site de clivage à la protéase ubiquiste, et dans lequel la séquence nucléotidique de codage pour au moins une des protéines non structurelles accessoires codées par le gène P est modifiée par la deuxième modification génétique de telle manière qu'au moins une des protéines non structurelles accessoires est détruite sur le plan fonctionnel.

2. Virus Sendai génétiquement modifié selon la revendication 1, **caractérisé en ce que** le site de clivage à la protéase ubiquiste remplacé est issu du gène F du virus de la maladie de Newcastle.

3. Virus Sendai génétiquement modifié selon la revendication 1, **caractérisé en ce que** le site de clivage à la protéase de SeV-WT présente la séquence d'acides aminés VPQSR (SEQ ID n° 5) et/ou le site de clivage à la protéase ubiquiste présente la séquence d'acides aminés RRQKR (SEQ ID n° 6).

4. Virus Sendai génétiquement modifié selon l'une quelconque des revendications précédentes, **caractérisé en ce qu'**au moins une des protéines non structurelles accessoires n'est pas transcriptible.

5. Virus Sendai génétiquement modifié selon l'une quelconque des revendications précédentes, **caractérisé en ce que** le codon START est détruit sur le plan fonctionnel dans au moins une des protéines non structurelles accessoires.

6. Virus Sendai génétiquement modifié selon la revendication 1, **caractérisé en ce que** les protéines non structurelles accessoires sont choisies parmi le groupe constitué de : C', C, Y1, Y2, V et W.

7. Virus Sendai génétiquement modifié selon la revendication 6, **caractérisé en ce qu'**au moins les protéines C (C et/ou C') et Y (Y1 et/ou Y2) sont modifiées, de préférence sont détruites sur le plan fonctionnel, par la deuxième modification génétique.

8. Virus Sendai génétiquement modifié selon la revendication 6, **caractérisé en ce qu'**au moins les protéines C (C et/ou C'), V et W sont modifiées, de préférence sont détruites sur le plan fonctionnel, par la deuxième modification génétique.

9. Virus Sendai génétiquement modifié selon la revendication 6, **caractérisé en ce qu'**au moins les protéines C (C et/ou C'), V, W et Y (Y1 et/ou Y2) sont modifiées, de préférence sont détruites sur le plan fonctionnel, par la deuxième modification génétique.

10. Virus Sendai génétiquement modifié selon l'une quelconque des revendications précédentes, qui présente par rapport au type sauvage (WT) au moins un transgène, lequel est un gène induisant ou immunostimulant de préférence le gène suicide ou une autre apoptose.

11. Composition pharmaceutique, qui présente le virus Sendai génétiquement modifié selon l'une quelconque des revendications 1 à 10 et un excipient pharmaceutiquement acceptable.

12. Utilisation d'un virus Sendai génétiquement modifié pour fabriquer un médicament pour le traitement thérapeutique et/ou prophylactique d'une tumeur, de préférence d'une tumeur solide, par ailleurs de préférence d'un carcinome hépatocellulaire, **caractérisée en ce que** le virus Sendai génétiquement modifié est le virus Sendai génétiquement modifié selon l'une quelconque des revendications 1 à 10.

13. Procédé pour fabriquer une composition pharmaceutique pour le traitement thérapeutique et/ou prophylactique d'une tumeur, de préférence d'une tumeur solide, par ailleurs de préférence d'un carcinome hépatocellulaire, qui présente des étapes suivantes :
(1) la fourniture du virus Sendai génétiquement modifié selon l'une quelconque des revendications 1 à 10 et
(2) la formulation du virus Sendai génétiquement modifié dans un excipient acceptable.
